# EUROPÄISCHE PATENTSCHRIFT

(11) **EP 1 909 727 B1**
(45) Veröffentlichungstag und Bekanntmachung des Hinweises auf die Patenterteilung: **10.12.2014**
(21) Anmeldenummer: 06762502.0
(22) Anmeldetag: 08.07.2006
(51) Int. Cl.: A61F 13/56, A61F 13/64

(54) **INKONTINENZARTIKEL**
INCONTINENCE PRODUCT
ARTICLE POUR L'ENURESIE

(30) Priorität: 29.07.2005 DE 102005035544
(43) Veröffentlichungstag der Anmeldung: 16.04.2008
(73) Patentinhaber: Paul Hartmann AG, 89522 Heidenheim (DE)
(72) Erfinder: ECKSTEIN, Axel, 89522 Heidenheim (DE); GRÖNER, Rudolf, 89555 Söhnstetten (DE); KESSELMEIER, Rüdiger, 89542 Herbrechtingen (DE); KOCH, Christian, 89429 Bachhagel (DE)
(86) Internationale Anmeldenummer: PCT/EP2006/006697
(87) Internationale Veröffentlichungsnummer: WO 2007/014623

(56) Entgegenhaltungen:
- EP-A- 1 661 540
- EP-A2- 1 269 949
- WO-A-2005/102241
- US-A1- 2001 034 512
- US-A1- 2002 045 881
- US-A1- 2004 023 341

## Beschreibung

Die vorliegende Erfindung betrifft einen absorbierenden Inkontinenzartikel gemäß Anspruch 1 insbesondere für inkontinente Erwachsene, mit einem Hauptteil, bestehend aus einem Vorderbereich, einem Rückbereich und einem in Längsrichtung dazwischen liegenden zwischen den Beinen eines Benutzers zu liegen kommenden Schrittbereich, wobei der Hauptteil einen Saugkörper umfasst, und mit an den Rückbereich und an den Vorderbereich angrenzenden ersten und zweiten Materialabschnitten, welche sich in Querrichtung oder Hüftumfangsrichtung über seitliche Längsränder des Hauptteils hinaus erstrecken und den Vorderbereich und den Rückbereich im angelegten Zustand des Artikels miteinander verbinden.

Bei derartigen Inkontinenzartikeln können die erwähnten, insbesondere nur an den Rückbereich angrenzenden Materialabschnitte aus einem anderen Material gebildet sein als der Hauptteil oder eine Komponente des Hauptteils, beispielsweise ein flüssigkeitsundurchlässiges Backsheet oder ein flüssigkeitsdurchlässiges-Topsheet darstellen. Beispielsweise können die Seitenteile bildenden Materialabschnitte, die häufig auch als "Ohren" des Inkontinenzartikels bezeichnet werden, atmungsaktiv, insbesondere luft- oder wasserdampfdurchlässig ausgebildet werden, wohingegen der Hauptteil, der häufig auch als Chassis bezeichnet wird, flüssigkeitsundurchlässig, insbesondere feuchtigkeitsundurchlässig ausgeführt sein kann. Zum Schließen des Inkontinenzartikels werden die vorzugsweise unlösbar am Rückbereich angefügten Seitenteile bildenden Materialabschnitte auf die Bauchseite des Benutzers geschlagen und dort mit dem Hauptteil, vorzugsweise mit der körperabgewandten Außenseite des Vorderbereichs des Hauptteils lösbar verbunden. Häufig kommen hierfür mechanisch wirkende oder klebend wirkende Verschlusselemente an den Seitenteilen des Inkontinenzartikels zum Einsatz, die dann mit entsprechend ausgebildeten Auftreffbereichen im Vorderbereich des Hauptteils zusammenwirken.

EP 1 269 949 A2 zeigt keinen Inkontinenzartikel, bei dem die von einem Rückbereich vorstehenden Seitenteile an dem Vorderbereich festgelegt werden, sondern eine so genannte Gürtelwindel, bei der die vom Rückbereich vorstehenden Seitenteile zur Bildung einer in Umfangsrichtung durchgehend geschlossenen Hüftöffnung miteinander verbunden werden. Die den Hüftgürtel bildenden seitlich vom Rückbereich vorstehenden Seitenteile sind Z-förmig aufeinandergefaltet und jeweils in dieser Konfiguration insbesondere durch einen unmittelbar nach der Herstellung der Windel an Klebkraft verlierenden Klebstoff aneinander fixiert.

Der vorliegenden Erfindung liegt die Aufgabe zugrunde, ausgehend von einem absorbierenden Inkontinenzartikel der eingangs beschriebenen Art mit verhältnismäßig breiten an den Hauptteil angefügten Materialabschnitten einerseits die Handhabung dieser Materialabschnitte während der Herstellung in einer schnelllaufenden Maschine zu verbessern und andererseits die Handhabung des Inkontinenzartikels bei der Ingebrauchnahme durch den Benutzer oder durch Pflegepersonen möglichst bedienerfreundlich und geschützt vor Verunreinigungen auszugestalten.

Diese Aufgabe wird erfindungsgemäß gelöst durch einen absorbierenden Inkontinenzartikel der eingangs genannten Art, wobei die Materialabschnitte vor Ingebrauchnahme des zusammengefalteten Artikels nach innen um eine in Längsrichtung verlaufende Einschlagachse auf die körperzugewandte Seite des Hauptteils eingeschlagen sind um eine übereinander eingeschlagene Anordnung zu bilden, derart, dass der zweite Materialabschnitt zumindest bereichsweise unterhalb des ersten Materialabschnittes zu liegen kommt und diese eingeschlagene Anordnung an einer ersten Fügestelle lösbar fixiert ist.

Währen bei Gürtelwindeln der Hüftgürtel verhältnismäßig schmal ausgebildet ist, werden absorbierende Inkontinenzartikel der eingangs genannten Art mit recht breiten seitlichen Materialabschnitten gewünscht. Mit der vorliegenden Erfindung wurde ein solcher Inkontinenzartikel geschaffen, bei dem die Anordnung der übereinander eingeschlagenen Materialabschnitte lösbar fixiert ist, so dass sie in der schnelllaufenden Herstellungsmaschine in der eingeschlagenen Anordnung gehalten wird, die Materialabschnitte somit nicht flattern können. Auch beim Entnehmen aus einer Umverpackung und beim Vorbereiten zum Anlegen des Hygieneartikels erweist sich die lösbare Fixierung der übereinander eingeschlagenen Anordnung der beiden Materialabschnitte als vorteilhaft, da die eingeschlagene Anordnung einen sicheren Kontaminationsschutz der körperzugewandten Seite, insbesondere des Topsheets des Inkontinenzartikels darstellt.

Die vorerwähnte lösbare Fixierung der übereinander eingeschlagenen Materialabschnitte kann beispielsweise durch ein Klebemittel insbesondere durch ein Heißschmelzklebemittel, weiter insbesondere durch ein Heißschmelzklebemittel mit permanenter Klebekraft erreicht werden. Als besonders vorteilhaft haben sich Schmelzhaftkleber erwiesen, die eine geringe Anfassklebrigkeit (Tack) aufweisen. Dies verhindert, dass das Klebemittel nach dem Öffnen der Fügestelle in unkontrollierter Weise beim oder nach dem Anlegen des Inkontinenzartikels an den Benutzer erneut mit weiteren Komponenten des Inkontinenzartikels oder der Wäsche oder der Haut des Benutzers verklebt. Als besonders vorteilhaft haben sich Heißschmelzklebemittel mit einer Viskosität gemessen bei 160 °C nach ASTM D3236 (von 1988) von höchstens 3000 mPa s, insbesondere von höchstens 2500 mPa s, weiter insbesondere von höchstens 2000 mPa s und weiter insbesondere von mindestens 1000 mPa s erwiesen. Heißschmelzklebemittel, die ein Polyolefin, insbesondere ein Polyolefin-Copolymer, weiter insbesondere ein Polyolefin-Copolymer und ein Polyisobutylen sowie einen Tackifier enthalten, erweisen sich als besonders zweckmäßig. Besonders bevorzugt enthalten diese Heißschmelzklebemittel außerdem ein Wachs als Plasticiser. Zu beziehen ist ein geeigneter Schmelzhaftklebstoff beispielsweise unter der Bezeichnung Technomelt HM5432 bei der Firma Henkel KGaA, Henkelstraße 67, D-40191 Düsseldorf oder unter der Bezeichnung H 5116 bei der Firma Bostik Nederland B.V., Zeggefeld 10, NL-4705 RP Roosendaal.

In einer alternativen Ausführungsform der Erfindung kann die vorerwähnte lösbare Fixierung durch kalte Verprägung, oder durch Verprägung unter Anwendung von Temperatur (Thermoverschweißung) oder durch Vernadelung, insbesondere Heißvernadelung, oder durch Ultraschallverschweißung oder Laserverschweißung oder durch ähnliche gleichwirkende Fügemethoden erreicht werden.

Es erweist sich als vorteilhaft, wenn ein Teilabschnitt des ersten Materialabschnittes an der ersten Fügestelle oder an ersten Fügestellen an einem Teilabschnitt des zweiten Materialabschnittes lösbar fixiert ist. Da erster und zweiter Materialabschnitt üblicherweise aus identischen Materialien gebildet werden ist hierdurch die Auswahl des Fügeverfahrens erleichtert.

Es kann sich aber als ebenso vorteilhaft erweisen, wenn ein Teilabschnitt des ersten Materialabschnittes an der ersten Fügestelle oder ersten Fügestellen an der körperabgewandten Oberfläche des Hauptteils, insbesondere in unmittelbarer Nähe des ersten seitlichen Längsrandes des Hauptteils lösbar fixiert ist, was insbesondere dann vorteilhaft ist, wenn die in Längsrichtung verlaufende Einschlagachse zumindest abschnittsweise innerhalb des Hauptteils verläuft. In unmittelbarer Nähe bedeutet, dass die erste Fügestelle oder die ersten Fügestellen von dem ersten seitlichen Längsrand des Hauptteils insbesondere höchstens 7 cm, weiter insbesondere höchstens 4 cm, weiter insbesondere höchstens 3 cm beabstandet sind. Hierdurch kann sicher vermieden werden, dass die Fügestelle oder die Fügestellen die Funktion des zweiten Materialabschnittes beeinträchtigen. Im Falle der Verwendung eines Klebemittels kann zum Beispiel hierdurch die Gefahr eines Kleberdurchschlages durch ein den zweiten Materialabschnitt bildendes poröses Material vermieden werden. Es erweist sich außerdem als vorteilhaft, die erste Fügestelle oder Fügestellen in einem unteren Bereich des ersten Materialabschnittes vorzusehen. Unter einem unteren Bereich des ersten Materialabschnittes wird im Rahmen dieser Erfindung ein Bereich verstanden, der in Längsrichtung des Inkontinenzartikels betrachtet näher zum unteren Querrand des ersten Materialabschnittes als zum jeweiligen einen Teil des Hüftöffnungsrandes des Inkontinenzartikels bildenden oberen Querrand des ersten Materialabschnittes angeordnet ist.

Die erste Fügestelle oder die ersten Fügestellen umfassen bevorzugt eine oder mehrere punktförmige Klebemittelstellen. In einer besonders bevorzugten Ausführungsform umfasst die erste Fügestelle zumindest eine kontinuierliche oder diskontinuierliche Klebemittelspur, insbesondere eine Heißschmelzklebemittelspur, wobei sich die Klebemittelspur in Längsrichtung über die gesamte Abmessung des ersten Materialabschnittes also über dessen gesamte Länge erstrecken kann. In einer besonders bevorzugten Ausführungsform beträgt die Länge der Klebemittelspur 10-120 mm, insbesondere 20-80 mm und weiter insbesondere 30-60 mm. Die Breite der Klebemittelspur, also deren Erstreckung in Querrichtung beträgt vorzugsweise 1-20 mm, insbesondere 1,5-15 mm und weiter insbesondere 2-10 mm.

In Weiterbildung der Erfindung erweist es sich als vorteilhaft, wenn die ersten und zweiten Materialabschnitte, insbesondere die des Rückbereiches, weiter insbesondere die des Rückbereiches und des Vorderbereiches an den Windelhauptteil als separate Elemente angefügt sind. Hierdurch kann durch Einsatz unterschiedlicher Materialien in Windelhauptteil und ersten und zweiten Materialabschnitten, die Windel mit Zonen unterschiedlicher Eigenschaften wie insbesondere Atmungsaktivität oder Luftdurchlässigkeit oder Festigkeit oder Weichheit oder Elastizität ausgebildet werden.

Es erweist sich in Weiterbildung der Erfindung als besonders vorteilhaft, wenn die Materialabschnitte ihrerseits wenigstens um eine in Längsrichtung verlaufende Falzlinie auf sich selbst gefaltet sind. Besonders vorteilhaft ist hierbei, wenn aufeinander gefaltete und flächenhaft aneinander liegende Teilabschnitte der Materialabschnitte in dieser gefalteten Konfiguration an zweiten Fügestellen lösbar aneinander fixiert sind. Hierdurch wird sichergestellt, dass die Materialabschnitte jeweils auch dann noch in geordneter Konfiguration vorliegen, wenn die erste, die übereinander eingeschlagene Anordnung haltende Fügestelle oder die ersten Fügestellen gelöst wurden. Außerdem ist im Zuge der Herstellung der Windel das Einschlagen der Materialabschnitte nach innen erleichtert.

Es erweist sich in Weiterbildung der Erfindung als besonders vorteilhaft, wenn an einem in Querrichtung das freie Ende des Materialabschnitts bildenden Teilabschnitt eines jeweiligen so gefalteten Materialabschnitts ein Anfassbereich zum Entfalten des Materialabschnitts vorgesehen ist. Dieser Anfassbereich kann im einfachsten Fall von einem Längsseitenrandabschnitt des erwähnten Teilabschnitts gebildet sein, der mit den Fingern eines Benutzers ergreifbar ist. Es wäre auch denkbar, dass an dem betreffenden Teilabschnitt ein separates manuell ergreifbares Anfasselement vorgesehen ist, was jedoch einen zusätzlichen herstellungstechnischen Aufwand bedeuten würde.

Des Weiteren erweist es sich für die Handhabung als zweckmäßig, dass der betreffende Teilabschnitt in dem Anfassbereich keine zweiten Fügestellen aufweist, dass er dort also nicht an weitere Teilabschnitte oder an den Hauptteil in einer Weise fixiert oder gefügt ist, die ein Ergreifen mit den Fingern des Benutzers behindern oder erschweren würde. Es wäre jedoch auch denkbar, dass der Benutzer mit seinen Fingern zwischen einzelne zweite Fügestellen eingreift oder hindurchgreift, um den Anfassbereich zu erfassen und dann durch Ziehen den Materialabschnitt unter Lösung der zweiten Fügestellen zu entfalten.

In diesem Zusammenhang erweist es sich nach einer besonders vorteilhaften und bevorzugten Weiterbildung des erfindungsgemäßen Inkontinenzartikels als vorteilhaft, dass die lösbare Fixierung an sämtlichen zweiten Fügestellen beim Entfalten durch einmaliges Ziehen an dem Anfassbereich der jeweiligen Materialabschnitte auftrennbar ist. Hierdurch wird die Handhabung weiter vereinfacht und der Inkontinenzartikel wird hierdurch noch bedienungsfreundlicher, gerade was die Anwendung bei stark pflegebedürftigen Personen betrifft.

Die vorerwähnte durch einmaliges Ziehen an einem Anfassbereich, also durch eine einzige Zugbewegung, erreichbare vollständige Entfaltung der gefalteten Materialabschnitte bedeutet, dass der Benutzer nicht mehrfach ruckartig an einem jeweiligen Materialabschnitt ziehen oder gar zerren muss, bis sämtliche Fügestellen zwischen den Teilabschnitten des Materialabschnitts und gegebenenfalls auch zum Hauptteil des Inkontinenzartikels gelöst werden.

In einer noch weiter bevorzugten Weiterbildung der Erfindung ist sowohl die lösbare Fixierung an sämtlichen ersten Fügestellen der eingeschlagenen Anordnung als auch die lösbare Fixierung an sämtlichen zweiten Fügestellen des ersten Materialabschnittes beim Entfalten durch einmaliges Ziehen an einem Anfassbereich des ersten Materialabschnittes auftrennbar.

Im einfachsten Fall ist ein jeweiliger Materialabschnitt um eine Falzlinie auf sich selbst gefaltet, so dass zwei Teilabschnitte aufeinander liegen bzw. gegeneinander anliegen. Vorzugsweise ist der Materialabschnitt aber um wenigstens zwei Falzlinien auf sich selbst gefaltet, so dass eine im Schnitt Z-förmige Konfiguration entsteht. Nach einer weiteren bevorzugten Ausführungsform sind die Materialabschnitte um drei Falzlinien auf sich selbst gefaltet. Nach einer weiteren bevorzugten Ausführungsform sind die Materialabschnitte um vier Falzlinien auf sich selbst gefaltet.

Nach einer weiteren bevorzugten Ausführungsform des erfindungsgemäßen Inkontinenzartikels sind die jeweiligen Anfassbereiche vor der Entfaltung der Materialabschnitte in Querrichtung nach außen gewandt, also voneinander und von einer Längsmittelachse des auf einer ebenen Unterlage ausgebreiteten Windelhauptteils voneinander weggewandt, so dass sie auf bequeme Weise mit der linken Hand eines Benutzers von links und mit der rechten Hand von rechts ergreifbar sind.

Die lösbare Fixierung der aufeinander gefalteten Teilabschnitte der Materialabschnitte aneinander und möglicherweise auch mit dem Hauptteil ist vorzugsweise durch mehrere im Wesentlichen punktförmige zweite Fügestellen ausgebildet. Eine punktförmige Fügestelle der vorstehend erwähnten Art bedeutet, dass die Fügestelle eine Fläche (in Projektion auf die X-Y-Ebene des Hauptteils von weniger als 5 mm², insbesondere von weniger als 2 mm² und weiter insbesondere von weniger als 1 mm² aufweist. Die zweiten Fügestellen müssen nicht streng punkt- oder kreisförmig sein. Denkbar und vorteilhaft sind auch von der Punkt- oder Kreisform abweichende Formen wie Dreieck-, Viereck-, Vieleck-, oder Ovalformen. Vorzugsweise ist die lösbare Fixierung der aufeinander gefalteten Teilabschnitte der Materialabschnitte aneinander durch thermisch oder durch Ultraschall erzeugte, vorzugsweise punktförmige zweite Fügestellen ausgebildet.

Es wurde erfindungsgemäß erkannt, dass die Anzahl, die Verteilung oder der Flächenanteil der zweiten Fügestellen oder die Haftkraft der lösbar aneinander gefügten Teilabschnitte so gewählt werden kann, dass die lösbare Fixierung an sämtlichen zweiten Fügestellen beim Entfalten durch einmaliges Ziehen an dem jeweiligen Anfassbereich der Materialabschnitte auftrennbar ist. Dies kann in vorteilhafter Weise dadurch unterstützt werden, dass die Anzahl oder der Flächenanteil der zweiten Fügestellen oder die Haftkraft der lösbar aneinander gefügten Teilabschnitte mit der Entfernung von dem Anfassbereich des Materialabschnitts abnimmt. Es wurde erfindungsgemäß erkannt, dass je weiter ein Bereich der aufeinander gefalteten Teilabschnitte der Materialabschnitte von dem Anfassbereich entfernt ist, desto geringer die Stärke der Fixierung der Teilabschnitte aneinander sein sollte, um eine Lösung sämtlicher zweiten Fügestellen oder -bereiche durch einmaliges Ziehen an dem jeweiligen Anfassbereich der Materialabschnitte, also durch eine einmalige Entfaltungsbewegung, zu erreichen. Es wurde demzufolge auch erkannt, dass in der Nähe des Anfassbereichs die lösbare Fixierung der aufeinander gefalteten Teilabschnitte problemlos den Anforderungen entsprechend stark ausgebildet werden kann. So kann ein sicherer Transport der vorzugsweise schon vor oder in der schnelllaufenden Windelherstellungsmaschine gefalteten Flachmaterialbahnen gewährleistet werden, ohne dass vom Hauptteil des Inkontinenzartikels seitlich vorstehende Materialabschnitte flattern oder aufeinander gefaltete Teilabschnitte innerhalb der Faltung verschoben werden. Es ergibt sich auch später bei der Faltung des gesamten Produkts, insbesondere bei Vorsehung der übereinander eingeschlagenen Anordnung der Materialabschnitte ein sauberes Erscheinungsbild.

In weiterer Ausbildung der Erfindung erweist es sich als vorteilhaft, wenn in einem Umkreis von 1,5 cm von einem vom Anfassbereich in Ebenenrichtung der gefalteten Konfiguration am weitesten entfernten Punkt der vorzugsweise am weitesten entfernten Falzlinie die aneinander anliegenden Teilabschnitte nicht durch zweite Fügestellen aneinander gefügt sind. Bei diesem am weitesten entfernten Punkt wird es sich um einen am freien Rand der Materialabschnitte liegenden Punkt der Falzlinie handeln. Doch auch über die gesamte Erstreckung der betrachteten insbesondere am weitesten vom Anfassbereich entfernten Falzlinie erweist es sich als vorteilhaft, wenn in einem Abstand von 5 mm, insbesondere von 8 mm und weiter insbesondere von 10 mm von dieser Falzlinie entfernt die aneinander liegenden Teilabschnitte nicht aneinander gefügt sind.

Es erweist sich des Weiteren als vorteilhaft, wenn die flächenhafte Erstreckung der aufeinander gefalteten und aneinander anliegenden Teilabschnitte (gedanklich) durch eine in Längsrichtung verlaufende Gerade in zwei annähernd gleiche Hälften teilbar ist und wenn die Anzahl oder der Flächenanteil der zweiten Fügestellen oder -bereiche oder die Haftkraft der lösbar aneinander gefügten Teilabschnitte in diesen zwei Hälften unterschiedlich ist. Es erweist sich dabei als besonders vorteilhaft, wenn die Anzahl oder der Flächenanteil der zweiten Fügestellen oder die Haftkraft der lösbar aneinander gefügten Teilabschnitte in der dem Anfassbereich in Querrichtung zugewandten Hälfte, also der daran angrenzenden Hälfte, größer ist als in der dem Anfassbereich in Querrichtung abgewandten Hälfte.

Die dem Hauptteil des Inkontinenzartikels angefügten Materialabschnitte sind, wie eingangs bereits erwähnt, breiter (quer zur Hüftumfangsrichtung) ausgebildet als dies bei typischen Gürtelwindeln der Fall ist. Die Breite, also die Erstreckung eines Materialabschnitts in Längsrichtung des Hygieneartikels beträgt im Bereich der Anfügung an den Hauptteil vorzugsweise wenigstens 10 cm, insbesondere wenigstens 14 cm, insbesondere wenigstens 18 cm und weiter insbesondere wenigstens 22 cm.

Die Erstreckung eines an den Hauptteil angefügten Materialabschnitts im entfalteten Zustand in Querrichtung über den Längsrand des Hauptteils hinaus, was der Hüftumfangsrichtung im angelegten Zustand entspricht, beträgt wenigstens 10 cm, insbesondere wenigstens 15 cm, und weiter insbesondere wenigstens 18 cm. Sie beträgt vorzugsweise höchstens 35 cm, insbesondere höchstens 30 cm und weiter insbesondere höchstens 27 cm. Es ist auch denkbar und für einzelne Inkontinenzartikel vorteilhaft, wenn sowohl im Vorderbereich als auch im Rückbereich solche vom Hauptteil in Querrichtung vorstehende Materialabschnitte angefügt sind. Es erweist sich solchenfalls als vorteilhaft, wenn alle derartigen Materialabschnitte des Inkontinenzartikels im erfindungsgemäßen Sinne gefaltet und lösbar fixiert sind.

Zum Schließen des Inkontinenzartikels im angelegten Zustand an einen Benutzer weisen die Materialabschnitte Verschlusselemente auf, die mechanisch haftend oder klebend ausgebildet sein können, und die vorzugsweise ihrerseits in einer gefalteten, zum Gebrauch entfaltbaren Konfiguration an den Materialabschnitten angeordnet sind. Die Materialabschnitte im Rückbereich weisen solche Verschlusselemente auf, die mit einem Auftreffbereich am Windelhauptteil lösbar haftend oder klebend zusammenwirken.

Die an den Hauptteil angefügten Materialabschnitte sind vorzugsweise aus einem Vliesstoff gebildet, insbesondere und vorzugsweise können Spunbond-Materialien (S) oder Spunbond-Meltblown-Materialien (SM), oder beidseitig mit Spunbond-Materialien versehene Meltblown-Schichten (SMS) oder auch kardierte Vliesmaterialien Verwendung finden. Auch Vliesstofflaminate, also insbesondere zweilagige, dreilagige oder mehrlagige Kombinationen der vorgenannten Vliesstoffe, können Verwendung finden. Die Verbindung der einzelnen Lagen kann durch an sich übliche und bekannte Verfahren, beispielsweise durch thermische Fügeverfahren (Verschweißung, insbesondere Laserverschweißung, hotmelt, airthrough) oder durch Ultraschallschweißverfahren erfolgen; auch die kalte Verpressung, Vernadelung, Vernähung oder Verklebung von Vliesstoffmaterialien ist denkbar. Auch die Verbindung mit textilen Geweben, Gewirken oder Gestricken, also mit eine textile Bindung im weitesten Sinne aufweisenden Materialien ist denkbar. Vorzugsweise werden die seitlich an den Hauptteil angefügten Materialabschnitte zumindest abschnittsweise atmungsaktiv ausgebildet, wobei insbesondere eine Mikroporosität als vorteilhaft angesehen wird, die sowohl einen Luftaustausch als auch eine Durchlässigkeit für Feuchtigkeit in Form von Wasserdampf gestattet. Die Materialabschnitte haben in vorteilhafter Weise ein Flächengewicht von 10 bis 150 g/m², insbesondere 20 - 100 g/m², weiter insbesondere 25 - 50 g/m².

Die an den Hauptteil angefügten Materialabschnitte können nach einer weiteren Ausführungsform der Erfindung auch so ausgebildet werden, dass sie weniger steif sind als der Hauptteil oder die chassisbildenden Materialien des Hauptteils, wie insbesondere das Backsheet oder ein aus Backsheet und Topsheet bestehendes Laminat des Hauptteils. Auf diese Weise kann ein hautfreundlicher Seitenabschnitt des Hygieneartikels erreicht werden, der vorzugsweise textil bzw. vliesartig anmutet und vom Benutzer als angenehm empfunden wird.

Es erweist sich nach einem an sich selbständigen Erfindungsgedanken als besonders vorteilhaft, dass die zweite Fügestelle oder die zweiten Fügestellen beim Entfalten der Materialabschnitte durch Ziehen an dem jeweiligen Anfassbereich eine über den Entfaltungsvorgang gemittelte Spitzenkraft von höchstens 2,5 N, insbesondere von höchstens 2,4 N, insbesondere von höchstens 2,3 N, insbesondere von höchstens 2,2 N, insbesondere von höchstens 2,1 N und weiter insbesondere von höchstens 2,0 N entgegensetzen. Ermittelt man während eines Entfaltungsvorgangs durch Ziehen an dem jeweiligen Anfassbereich die dabei in jedem Zeitpunkt auftretende Zugkraft und berücksichtigt diejenigen Kraftspitzen, die sich von unmittelbar angrenzenden, benachbarten Bereichen um wenigstens 0,5 N unterscheiden, so ist es möglich, sämtliche so ermittelten Kraftspitzen während eines Entfaltungsvorgangs zu mitteln, also eine über die Anzahl dieser Kraftspitzen gemittelte Spitzenkraft zu errechnen.

Berücksichtigt man nicht nur einen Entfaltungsvorgang, sondern sechs Entfaltungsvorgänge von sechs identisch gebildeten und gefalteten Materialabschnitten und mittelt die wie vorausgehend ermittelten gemittelten Spitzenkräfte über die sechs Entfaltungsvorgänge, so betragen diese vorzugsweise höchstens 2,3 N, insbesondere höchstens 2,0 N, insbesondere höchstens 1,8 N, insbesondere höchstens 1,7 N, insbesondere höchstens 1,6 N und weiter insbesondere höchstens 1,5 N.

Es erweist sich des Weiteren als besonders vorteilhaft, dass nach einem weiteren an sich unabhängigen Erfindungsgedanken die Materialabschnitte derart gefaltet und durch zweite Fügestellen vorfixiert sind, dass die beim Entfalten eines Materialabschnitts in einem Zug erforderliche Arbeit gemittelt über sechs Entfaltungsvorgänge höchstens 120 Nmm, insbesondere höchstens 115 Nmm, insbesondere höchstens 110 Nmm, insbesondere höchstens 105 Nmm, insbesondere höchstens 100 Nmm, insbesondere höchstens 95 Nmm, insbesondere höchstens 90 Nmm beträgt.

Es wird nachfolgend ein Test zur Bestimmung der beim Entfalten und damit zum Lösen der zweiten Fügestelle oder zweiten Fügestellen der auf sich selbst gefalteten Materialabschnitte zu überwindenden Kräfte und zur Prüfung des Entfaltens der Materialabschnitte in einem Zug beschrieben. Es wird unter Verwendung eines Zugprüfgeräts nach EN ISO 527-1 (April 1996) die in jedem Moment auftretende Kraft über den Öffnungsweg ermittelt und aufgezeichnet.

### Probenvorbereitung:

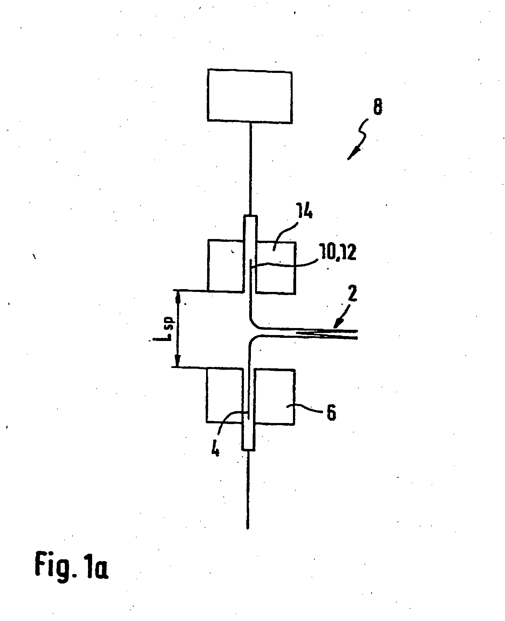

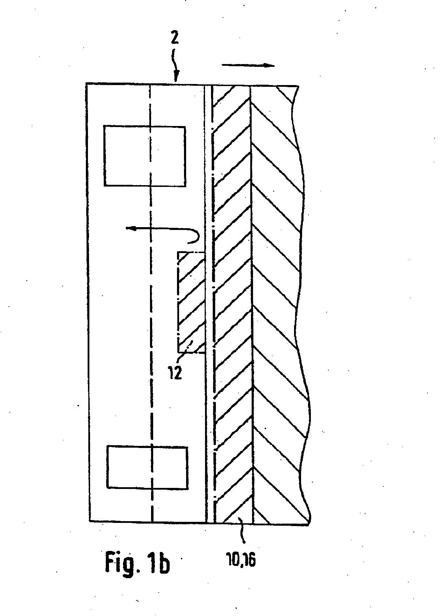

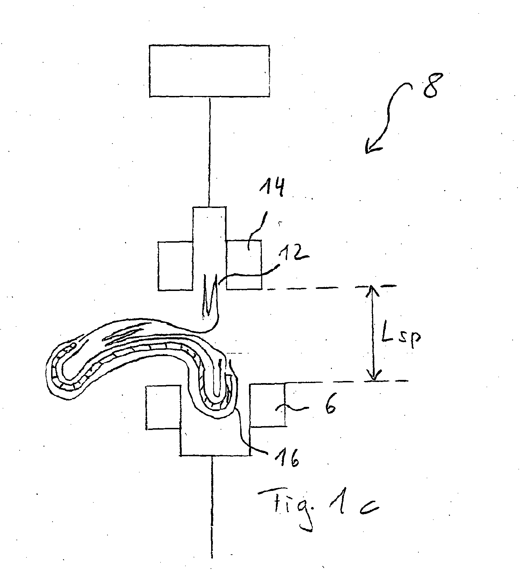

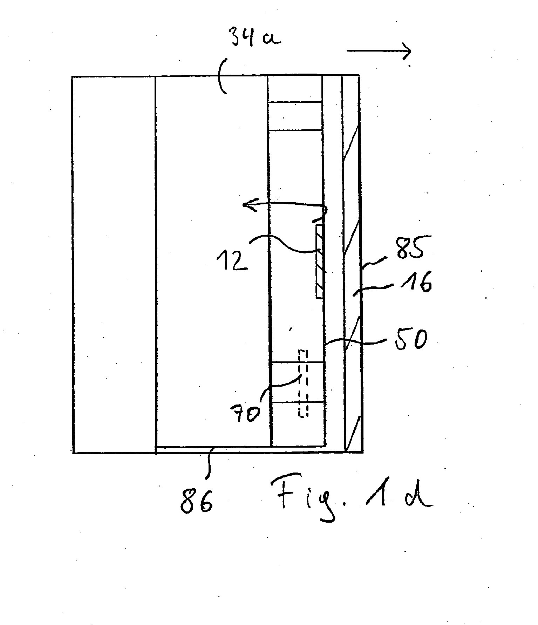

Zunächst wird bei einem Hygieneartikel die übereinander eingeschlagene Anordnung der Materialabschnitte durch Lösen der ersten Fügestellen aufgeschlagen. Von dem Hygieneartikel wird dann ein an den Hauptteil angefügter auf sich selbst gefalteter Materialabschnitt entlang eines Seitenlängsrandes des Hauptteils unter Zerstörung der Anfügung abgetrennt. Hierfür kann eine Klinge oder Schere Verwendung finden. Der den betreffenden Seitenteil bildende Materialabschnitt 2 wird gemäß Figuren 1 a und 1 b mit einem Längsseitenrand 4 an eine untere Klemme 6 des Zugprüfgeräts über seine gesamte Länge (in Längsrichtung des Inkontinenzartikels), mit der er zuvor an den Hauptteil angefügt war, fest eingespannt. Die untere Klemme 6 des Zugprüfgeräts 8 muss deshalb eine entsprechende Länge, zweckmäßigerweise eine Länge von 300 mm aufweisen. Am gegenüberliegenden freien Längsseitenrand 10 des abgetrennten Materialabschnitts 2, der dort einen Anfassbereich 12 bildet, wird die bewegbare Klemme 14 des Zugprüfgeräts 8 über eine Länge von 60 mm eingespannt. Bei einer Längserstreckung des Materialabschnitts von weniger als 160 mm wird der Materialabschnitt 2 am freien Längsseitenrand 10 über eine Länge von 30 mm an der beweglichen Klemme 14 eingespannt. Die nicht maßstabsgetreue Figur 1 b zeigt den Materialabschnitt 2 in der gefalteten Konfiguration, wobei der in die Klemme 6 einspannbare Bereich 16 am Längsseitenrand 10 und der in der Klemme 14 einspannbare Anfassbereich 12 schraffiert dargestellt ist. Die Pfeile deuten die Zugrichtung des Zugprüfgeräts an. In Figur 1a ist ferner die Einspannlänge Lₛₚ angedeutet.

Durch gesteuerte Bewegung dieser bewegbaren Klemme 14 wird ein Zugprüfversuch durchgeführt.

### Prüfparameter:

- Prüfgeschwindigkeit der bewegbaren Klemme: 300 mm/min
- Einspannlänge: 10 mm
- Messweg: Länge der Quererstreckung des entfalteten Materialabschnitts
- Vorkraft: 0,01 N.

### Auswertung:

Das Prüfresultat des Zugprüfversuchs wird als in dem Materialabschnitt aufgetretene und zwischen den Klemmen ermittelte Zugkraft gerundet auf zwei Dezimalstellen in N angegeben. Es wird ein Kraft/Wegdiagramm erstellt.

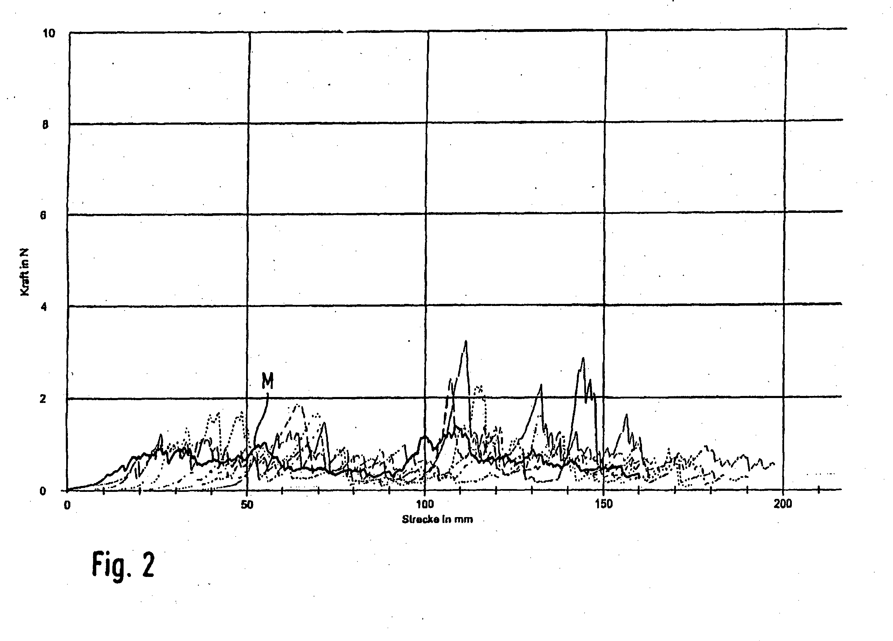

Ein solches Kraft/Wegdiagramm zeigt Figur 2. Es sind in Figur 2 die Ergebnisse von sechs Zugprüfversuchen und fett eine hieraus gemittelte Kurve M bei einem nachfolgend noch zu beschreibenden Inkontinenzartikel dargestellt. In der nachfolgenden Tabelle 1 sind jeweils die Spitzenkräfte Fmax und die für einen jeweiligen Entfaltungsvorgang ermittelten Mittelwerte von Fmax angegeben. Von einer Kraftspitze Fmax wurde ausgegangen, wenn sich diese um 0,5 N von einem benachbarten Kraftminimum unterschied. Sollte das Kraft/Wegediagramm des Ablösevorgangs lediglich eine einzige Kraftspitze aufweisen gilt somit: Spitzenkraft Fmax = Mittelwert Fmax.

In der Tabelle 1 außerdem angegeben ist die zum Öffnen erforderliche Arbeit in Nmm, die rechnerisch aus den ermittelten Zugkräften und dem Weg ermittelt wurde.

| Nr | Mittelwert Fₘₐₓ in N | Fₘₐₓ in N | W in Nmm |
|---|---|---|---|
| 1 | 1, 78 | 3,24 | 95,69 |
| 2 | 1,41 | 2,41 | 91,63 |
| 3 | 1,61 | 2,25 | 89,91 |
| 4 | 1,16 | 1,39 | 62,66 |
| 5 | 1,12 | 1,62 | 60,84 |
| 6 | 1,69 | 2,86 | 95,40 |

**Tabelle 1**

| | | | |
|---|---|---|---|
| X(n=6) | 1,46 | 2,29 | 82,69 |

Die Ergebnisse nach Figur 2 und der vorstehenden Tabelle 1 wurden ermittelt bei der Entfaltung von Materialabschnitten, die in einem Rückbereich eines erfindungsgemäßen Hygieneartikels vorgesehen waren, der nachfolgend noch beschrieben wird, wobei diese Materialabschnitte zugleich versteifende Verschlusselemente aufweisen.

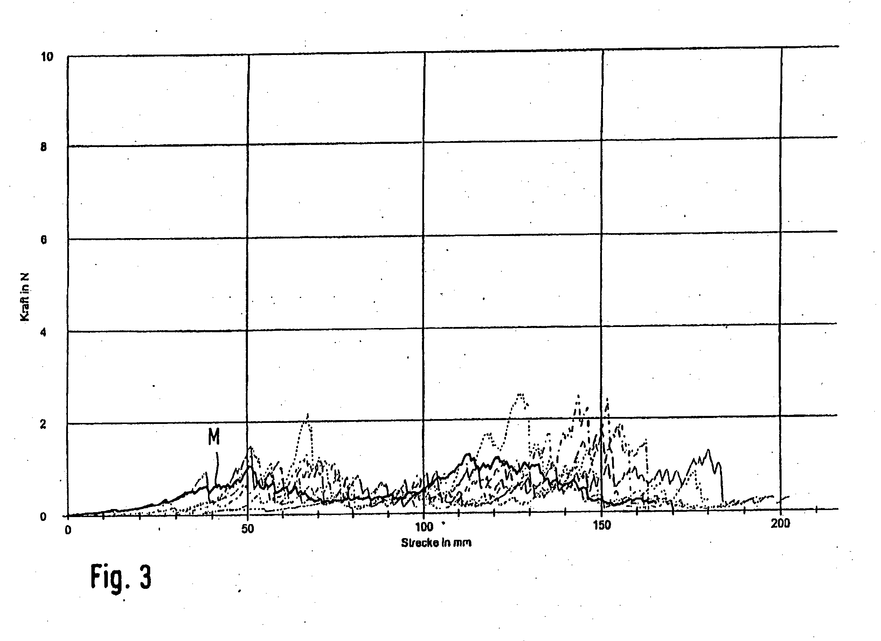

Entsprechende Messungen wurden an in einem Vorderbereich des Hygieneartikels vorgesehenen Materialabschnitten einer geringeren Längserstreckung und ohne versteifende Verschlusselemente vorgenommen. Die Ergebnisse sind in Figur 3 und in der nachfolgenden Tabelle 2 in entsprechender Weise dargestellt.

**Tabelle 2**

| Nr | Mittelwert Fₘₐₓ in N | Fₘₐₓ in N | W in Nmm |
|---|---|---|---|
| 1 | 1,05 | 1,48 | 64,67 |
| 2 | 1,20 | 1,77 | 73,73 |
| 3 | 1,52 | 2,54 | 105,45 |
| 4 | 1,73 | 2,49 | 93,93 |
| 5 | 1,32 | 1,88 | 59,04 |
| 6 | 1,10 | 1,30 | 66,19 |
| X(n=6) | 1,32 | 1,91 | 77,17 |

Es erweist sich nach einem an sich selbständigen Erfindungsgedanken als besonders vorteilhaft, dass beim Lösen der übereinander eingeschlagene Anordnung die erste Fügestelle oder der ersten Fügstellen eine über den Ablösungsvorgang gemittelte Spitzenkraft von höchstens 3,0 N, insbesondere von höchstens 2,0 N, insbesondere von höchstens 1,5 N und weiter insbesondere von höchstens 1,0 N entgegensetzen.

Berücksichtigt man nicht nur einen Ablösevorgang, sondern sechs Ablösevorgänge von sechs identisch gebildeten und eingeschlagenen Materialabschnitten und mittelt die wie vorausgehend ermittelten gemittelten Spitzenkräfte über die sechs Ablösevorgänge, so betragen diese vorzugsweise höchstens 2,5 N, insbesondere höchstens 2,0 N, insbesondere höchstens 1,5 N und weiter insbesondere höchstens 1,0 N.

Es erweist sich des Weiteren als besonders vorteilhaft, dass nach einem weiteren an sich unabhängigen Erfindungsgedanken die Materialabschnitte in der eingeschlagenen Anordnung durch eine erste Fügestelle oder durch erste Fügestellen derart vorfixiert sind, dass die beim Lösen der ersten Fügestelle oder ersten Fügestellen in einem Zug erforderliche Arbeit gemittelt über sechs Ablösevorgänge höchstens 50 Nmm, insbesondere höchstens 40 Nmm, insbesondere höchstens 30 Nmm und weiter insbesondere höchstens 20 Nmm beträgt.

In dem die Ablösekräfte und die zum Ablösen erforderliche Arbeit begrenzt ist, wird sichergestellt, dass die eingeschlagene Anordnung vom Benutzer ohne Schwierigkeiten, insbesondere ohne Beschädigung der jeweiligen Materialkomponenten gelöst werden kann.

Es wird nachfolgend ein Test zur Bestimmung der beim Lösen der die übereinander eingeschlagene Anordnung fixierenden ersten Fügestelle oder ersten Fügstellen zu überwindenden Kräfte beschrieben. Es wird unter Verwendung eines Zugprüfgeräts nach EN ISO 527-1 (April 1996) die in jedem Moment auftretende Kraft über den Öffnungsweg ermittelt und aufgezeichnet.

### Probenvorbereitung:

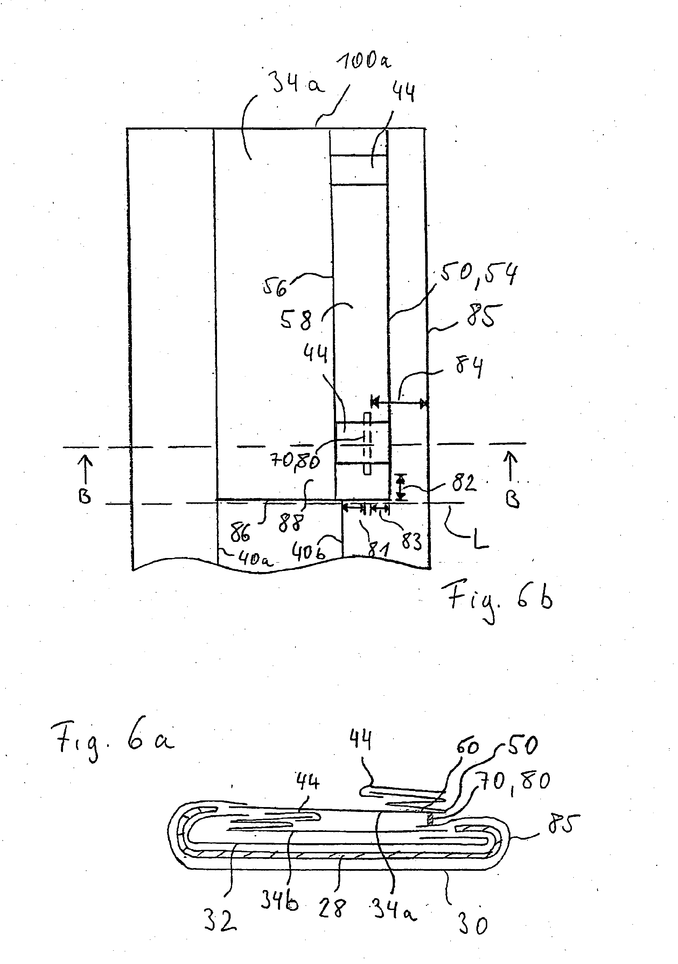

Zunächst wird bei einem Hygieneartikel mit in Figuren 6a und 6b dargestellter übereinander eingeschlagener Anordnung der Materialabschnitte der die übereinander geschlagene Anordnung aufweisende gesamte Vorder- oder Rückbereich 22, 24 durch einen Schnitt entlang einer Linie L in Querrichtung 38 des Hygieneartikels vorzugsweise unmittelbar unterhalb des unteren Querrandes 86 des ersten Materialabschnittes 34a, in jedem Fall aber unterhalb der ersten Fügestelle 70 abgetrennt. Hierfür kann eine Klinge oder Schere Verwendung finden.

Der betreffende Vorder- oder Rückbereich wird dann gemäß Figuren 1c und 1d an der Einschlagkante 85 an eine untere Klemme 6 des Zugprüfgeräts über einen seine gesamte Länge (in Längsrichtung des Inkontinenzartikels) umfassenden Bereich 16 mittig fest eingespannt. Die untere Klemme 6 des Zugprüfgeräts 8 muss deshalb eine entsprechende Länge, zweckmäßigerweise eine Länge von 300 mm aufweisen. An der Falzlinie 50, welche den Teilabschnitt 60, mit dem der erste Materialabschnitt 34a an der ersten Fügestelle 70 fixiert ist, begrenzt, wird die bewegbare Klemme 14 des Zugprüfgeräts 8 über eine Länge von 60 mm und über eine Tiefe von 5 mm in einem mit Bezugszeichen 12 bezeichneten Bereich mittig fest eingespannt. Die nicht maßstabsgetreue Figur 1d zeigt den Rückbereich in der eingeschlagenen Anordnung der Materialabschnitte, wobei der in die untere Klemme 6 einspannbare Bereich 16 an der Einschlagkante 85 und der in der oberen Klemme 14 einspannbare Bereich 12 schraffiert dargestellt sind. Die Pfeile deuten die Zugrichtung des Zugprüfgeräts an. In Figur 1c, in deren Ansicht die erste Fügestelle 70 nicht erkennbar und daher nicht dargestellt ist, ist ferner die Einspannlänge Lₛₚ angedeutet.

Durch gesteuerte Bewegung dieser bewegbaren Klemme 14 wird ein Zugprüfversuch durchgeführt.

### Prüfparameter:

- Prüfgeschwindigkeit der bewegbaren Klemme: 300 mm/min
- Einspannlänge: 50 mm
- Messweg: Strecke zur vollständigen Ablösung der ersten Fügestelle oder ersten Fügestellen der eingeschlagenen Anordnung
- Vorkraft: 0,2 N.

### Auswertung:

Das Prüfresultat des Zugprüfversuchs wird als in dem Vorder- oder Rückbereich aufgetretene und zwischen den Klemmen ermittelte Zugkraft gerundet auf zwei Dezimalstellen in N angegeben. Es wird ein Kraft/Wegdiagramm erstellt.

Wie bei der zuvor beschriebenen Prüfmethode zur Ermittlung der Öffnungskräfte eines auf sich selbst gefalteten Materialabschnittes werden die Spitzenkräfte Fmax und die für einen jeweiligen Ablösungsvorgang ermittelten Mittelwerte von Fmax angegeben. Von einer Kraftspitze Fmax wurde ausgegangen, wenn sich diese um 0,1 N von einem benachbarten Kraftminimum unterschied. Sollte das Kraft/Wegediagramm des Ablösevorgangs lediglich eine einzige Kraftspitze aufweisen gilt somit: Spitzenkraft Fmax = Mittelwert Fmax. In der nachfolgenden Tabelle 3 außerdem angegeben ist die zum Ablösen erforderliche Arbeit in Nmm, die rechnerisch aus den ermittelten Zugkräften und dem Weg ermittelt wurde.

**Tabelle 3**

| Nr | Mittelwert Fₘₐₓ In N | Fₘₐₓ in N | W in Nmm |
|---|---|---|---|
| 1 | 1,00 | 1,00 | 24 |
| 2 | 0,93 | 0,93 | 15 |
| 3 | 0,95 | 0,95 | 19 |
| 4 | 0,81 | 0,81 | 15 |
| 5 | 0,85 | 0,91 | 20 |
| 6 | 0,91 | 0,91 | 19 |
| X(n=6) | 0,91 | 0,92 | 18,7 |

In einem weiteren Erfindungsgedanken wird Schutz beansprucht für ein Verfahren zur Herstellung eines Inkontinenzartikels mit den Merkmalen der Ansprüche 40-42.

Weitere Merkmale, Einzelheiten und Vorteile der Erfindung ergeben sich aus den beigefügten Patentansprüchen, der zeichnerischen Darstellung und nachfolgenden Beschreibung einer bevorzugten Ausführungsform der Erfindung. In der Zeichnung zeigt:
Figur 1 a eine schematische Darstellung der Einspannung eines Materialabschnitts in ein Zugprüfgerät;
Figur 1b eine nicht maßstabsgetreue Darstellung eines Materialabschnitts in der auf sich selbst gefalteten Konfiguration
Figur 1c eine schematische Darstellung der Einspannung eines Rückbereiches eines Hygieneartikels mit zwei leporelloförmig auf sich selbst gefalteten und erfindungsgemäß nach innen zu einer eingeschlagenen Anordnung eingeschlagenen Materialabschnitten in ein Zugprüfgerät
Figur 1d eine nicht maßstabsgetreue Darstellung eines abgetrennten Rückbereiches eines Hygieneartikels mit zwei leporelloförmig auf sich selbst gefalteten und erfindungsgemäß nach innen zu einer eingeschlagenen Anordnung eingeschlagenen Materialabschnitten
Figuren 2,3 bei einem Zugprüfversuch ermittelte Kraft/Weg-Diagramme;
Figur 4 eine Draufsicht auf einen Hygieneartikel in schematischer Darstellung;
Figur 5 eine Schnittansicht des Hygieneartikels nach Figur 4 (A-A);
Figur 6a eine nicht maßstabsgetreue Schnittansicht mit zwei leporelloförmig auf sich selbst gefalteten und erfindungsgemäß nach innen zu einer eingeschlagenen Anordnung eingeschlagenen Materialabschnitten nach Figur 6b (B-B)
Figur 6b eine nicht maßstabsgetreue Teildraufsicht auf die körperzugewandte Seite des Rückbereiches eines Hygieneartikels mit zwei leporelloförmig auf sich selbst gefalteten und erfindungsgemäß nach innen zu einer eingeschlagenen Anordnung eingeschlagenen Materialabschnitten
Figur 7 eine vergrößerte Darstellung eines Materialabschnitts in auf sich selbst gefalteter Konfiguration;
Figur 8 eine schematische Schnittansicht eines auf sich selbst gefalteten Materialabschnitts.

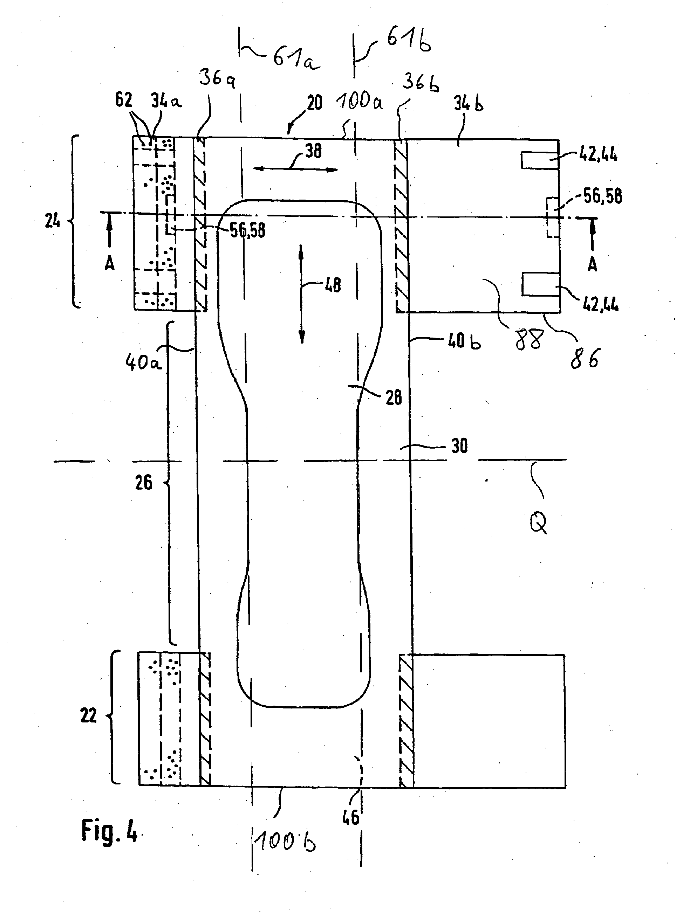

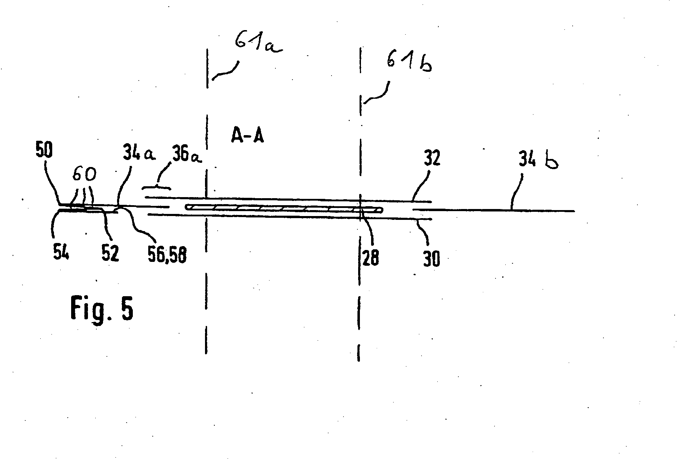

Der erfindungsgemäße Inkontinenzartikel ist in den Figuren 4 bis 8 schematisch dargestellt. Er umfasst einen insgesamt mit dem Bezugszeichen 20 bezeichneten Hauptteil, der häufig auch als Chassis bezeichnet wird. Der Hauptteil 20 umfasst einen Vorderbereich 22, einen Rückbereich 24 und einen dazwischen liegenden Schrittbereich 26, der zwischen den Beinen eines Benutzers zu liegen kommt, wenn der Hygieneartikel an einen Benutzer angelegt ist. Der Inkontinenzartikel weist weiter eine zentrale Querachse Q auf, welche den Inkontinenzartikel gedanklich in zwei in Längsrichtung gleich lange Abschnitte teilt. Der Hauptteil 20 umfasst einen Saugkörper 28, der zur Aufnahme und dauerhaften Speicherung von Körperflüssigkeiten in geeigneter Weise dimensioniert ist. Der Saugkörper ist von einer flüssigkeitsundurchlässigen Schicht 30 unterfangen, welche auch die äußere Sichtseite des Inkontinenzartikels bilden kann. Oberhalb des Saugkörpers 28 kann ein flüssigkeitsdurchlässiges Topsheet 32 vorgesehen sein, welches in der Schnittansicht der Figur 5 dargestellt ist.

Im Rückbereich 24 sind an einem ersten Seitenrandabschnitt 36a des Hauptteils 20 ein erster Seitenklappen oder Seitenabschnitte bildender Materialabschnitt 34a und an einem zweiten Seitenrandabschnitt 36b des Hauptteils 20 ein zweiter Seitenklappen oder Seitenabschnitte bildender Materialabschnitt 34b angefügt. Die Materialabschnitte 34a, 34b weisen eine rechteckförmige Kontur auf. Die äußeren Querränder von Hauptteil 20 und erstem 34a und zweitem Materialabschnitt 34b bilden den hinteren Hüftöffnungsrand 100a. Zur Bildung beidseitiger Seitenabschnitte wäre es aber auch denkbar, dass ein in Querrichtung 38 durchgehender Materialabschnitt vorgesehen ist, der beide Seitenabschnitte des Inkontinenzartikels bildet.

Figur 4 zeigt eine Sicht auf die körperzugewandte Seite des Inkontinenzartikels mit Materialabschnitten, deren eingeschlagene Anordnung gelöst und aufgeschlagen ist. Darüber hinaus ist in der Zeichnung der rechte, zweite Materialabschnitt 34b im vollständig entfalteten Zustand dargestellt. Er erstreckt sich in Querrichtung 38 über den betreffenden Längsrand 40b des Hauptteils 20 hinaus in Hüftumfangsrichtung. Der Materialabschnitt trägt zwei Verschlusselemente 42 in Form von auf sich selbst gefalteten und zum bestimmungsgemäßen Gebrauch entfaltbaren Verschlusstapes 44, die zum Schließen des Hygieneartikels mit einer Außenseite 46 des Vorderbereichs 22 des Hauptteils 20 und der Materialabschnitte im Vorderbereich 22 lösbar haftend zusammenwirken.

Der jeweilige Materialabschnitt 34a, 34b im Rückbereich 24 des Hauptteils ist, wie aus der Figur 4 oben links und der Schnittdarstellung nach Figur 5 ersichtlich, entlang dreier in Längsrichtung 48 erstreckter Falzlinien 50, 52, 54 leporelloförmig auf sich selbst gefaltet, wobei Teilabschnitte 60 aufeinander zu liegen kommen.

Ein freier Längsrandabschnitt 56 des jeweiligen Materialabschnitts 34a, 34b bildet einen Anfassbereich 58 zum manuellen Ergreifen des gefalteten Materialabschnitts 34, um diesen zu entfalten. Bei der Abgabe an den Letztverbraucher sind die jeweiligen Materialabschnitte 34a, 34b ausgehend von der Darstellung nach Figur 5 nach innen an jeweiligen zur Längsrichtung 48 parallelen Einschlagachsen 61 a, 61 b die in der dargestellten Ausführungsform innerhalb des Hauptteils 20 verlaufen, zu einer eingeschlagenen Anordnung geschlagen in die in Figur 6a dargestellte Position. In dieser Darstellung ist auch der zweite Materialabschnitt 34b in leporelloförmig gefalteter Konfiguration dargestellt. Man erkennt, dass der zweite Materialabschnitt 34b unterhalb des ersten Materialabschnitts 34a zu liegen kommt. Der erste Materialabschnitt 34a ist an einem Teilabschnitt 60 mittels einer ersten Fügestelle 70 an der die körperabgewandte Oberfläche des Hauptteils bildenden flüssigkeits-undurchlässigen Schicht 30 lösbar fixiert. Figur 6b zeigt eine Teildraufsicht auf die in Figur 6a dargestellte eingeschlagene Anordnung der Materialabschnitte 34a, 34b. Um die Übersichtlichkeit zu wahren sind mit Ausnahme der ersten Fügestelle 70 keine nicht sichtbaren Komponenten oder Falzlinien dargestellt. Die erste Fügestelle 70 wird durch eine sich in Längsrichtung 48 des Inkontinenzartikels über 40 mm und sich in Querrichtung 38 des Inkontinenzartikels über 3 mm erstreckende Heißschmelzklebemittelspur 80 gebildet. Der Abstand 81 der Klebemittelspur 80 zum zweiten Längsrand 40b des Hauptteils 20 beträgt 20 mm. Der Abstand 82 der Klebemittelspur 80 vom unteren Querrand 86 des ersten Materialabschnittes 34a beträgt lediglich 20 mm. Die Klebespur ist somit vollständig in einem unteren Bereich 88 des ersten Materialabschnittes 34a, also in einem Bereich des ersten Materialabschnittes 34a, der in Längsrichtung 48 betrachtet dichter zum unteren Querrand 86 des ersten Materialabschnittes 34a als zum entsprechenden hinteren Hüftöffnungsrand 100a angeordnet ist. Der Abstand 83 der Klebemittelspur 80 von der den Teilabschnitt 60 begrenzenden Falzlinie 50 des ersten Materialabschnittes beträgt 15 mm. Der Abstand 84 der Klebemittelspur 80 von der Einschlagkante 85 des zweiten Materialabschnittes 34b beträgt 40 mm. Erkennbar ist, dass die Klebemittelspur 80 direkt unterhalb der durch das Verschlusstape 44 gebildeten Projektionsfläche angeordnet sind. Dies hat den Vorteil, dass die Verschlussmittel gleichsam eine Anfasshilfe für den Benutzer zum Lösen der ersten Fügestelle 70, im dargestellten Fall zum Lösen der Klebemittelspur 80 und damit zur Aufhebung der Verbindung zwischen dem ersten Materialabschnitt 34a und derflüssigkeitsdurchlässigen Schicht 30 dienen kann.

Man erkennt außerdem, dass der jeweilige freie Längsrandabschnitt 56 und damit der Anfassbereich 58 der Materialabschnitte 34a, 34b von einer Längsmittelachse des Inkontinenzartikels weg nach außen gewandt ist, so dass bei auf einer Unterlage ausgebreitetem Hygieneartikel der Anfassbereich 58 des ersten Materialabschnitts 34a bequem mit der linken Hand eines Benutzers von links und nach Lösen der ersten Fügestelle 70 ein entsprechend positionierter Anfassbereich des zweiten Materialabschnitts 34b mit der rechten Hand des Benutzers von rechts ergriffen werden kann.

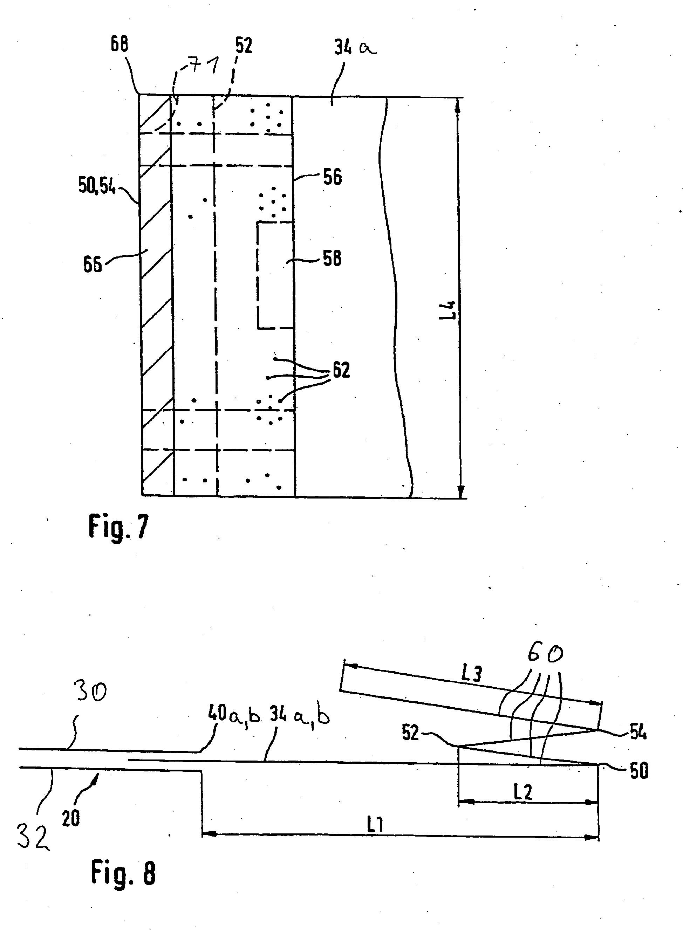

Die aufeinander gefalteten Teilabschnitte 60 weisen in einer ersten Ausführungsform keine zweiten Fügestellen auf. In der hier dargestellten Ausführungsform sind die gefalteten Teilabschnitte 60 der Materialabschnitte 34a, 34b hingegen in der gefalteten Konfiguration durch punktförmige durch Ultraschallschweißung erzeugte zweite Fügestellen 62 mit einem Durchmesser von 0,35 mm und einer Fläche von 0,0962 mm², die in Figur 4 angedeutet sind, lösbar aneinander fixiert. Es hat sich gezeigt, dass diese lösbare Fixierung so gestaltet sein kann, dass sich durch einmaliges Ziehen an dem jeweiligen Anfassbereich 58 der jeweilige Materialabschnitt 34a, 34b vollständig entfalten lässt, wobei alle zweiten Fügestellen 62 gelöst oder aufgetrennt werden. Hierbei erweist es sich als vorteilhaft, wenn in einem Abstand von wenigstens 5 mm, vorzugsweise von wenigstens 8 mm und weiter vorzugsweise von wenigstens 10 mm von den Falzlinien 50, 54 entfernt keine zweiten Fügestelle der Teilabschnitte 60 vorgesehen ist. Dieser fügestellenfreie Bereich ist in der Figur 7 mit Bezugszeichen 66 bezeichnet. Ausgehend von einem vom Anfassbereich 58 am weitesten entfernten Punkt 68 auf der Falzlinie 50, 54 ist in einem schematisch angedeuteten Umkreis 71 keine zweite Fügestelle vorgesehen. Diese sehr weit von dem Anfassbereich 58 entfernten und in der Nähe einer Falzlinie angeordneten Bereiche sind im Hinblick auf eine vollständige Entfaltung, d. h. eine Auftrennung sämtlicher zweiter Fügestellen besonders kritisch. Daher erweist es sich als vorteilhaft, wenn in diesen kritischen Bereichen nur wenige oder vorzugsweise keine zweiten Fügestellen vorgesehen sind, so dass die gefalteten Materialabschnitte in einem Zug, also ohne mehrfach Nachfassen oder ohne mehrfach ruckartig an dem Anfassbereich 58 ziehen zu müssen, lösbar sind bzw. entfaltbar sind.

Anhand der Figur 8 werden die Abmessungen der Teilabschnitte 60 des jeweiligen Materialabschnitts 34a, 34b deutlich. Die Erstreckung L1 in Querrichtung 38 vom jeweiligen Längsrand 40a, 40b des Hauptteils 20 bis zur Falzlinie 50 beträgt 90 mm. Die Erstreckung L2 zwischen den Falzlinien 50 - 52 - 54 beträgt jeweils 35 mm, und die Erstreckung L3 bis zum freien Ende beträgt 65 mm. Die Gesamterstreckung in Querrichtung des entfalteten Materialabschnitts beträgt daher in Querrichtung 38 225 mm. Die Längserstreckung L4 beträgt 260 mm (Fig. 7).

Wie aus Figur 4 ersichtlich ist, sind im Vorderbereich 22 des Hygieneartikels ebenfalls auf sich selbst gefaltete Materialabschnitte mit einer Längserstreckung L4 von nur 200 mm und einer Quererstreckung von 225 mm vorgesehen, die jedoch keine Verschlusselemente aufweisen, welche die Materialabschnitte versteifen und deshalb auch zum Lösen benachbart angeordneter zweiter Fügestellen aufgrund ihrer Steifigkeit beitragen. In unmittelbarer Nähe zu solchen versteifenden Verschlusselementen können nämlich zweite Fügestellen oder Fügebereiche vorgesehen werden, die aufgrund der Versteifung gut lösbar sind.

Wie bereits erwähnt, wurden die Messergebnisse nach Tabelle 1 und Figur 2 bei Entfaltungsvorgängen der Materialabschnitte 34a, 34b im Rückbereich 24 und die Messergebnisse nach Tabelle 2 und Figur 3 bei Entfaltungsvorgängen der Materialabschnitte im Vorderbereich 22 ermittelt. Die Messergebnisse nach Tabelle 3 wurden bei Ablösevorgängen der als Klebemittelspur 80 ausgebildeten ersten Fügestelle 70 der eingeschlagenen Anordnung der Materialabschnitte 34a, 34b des Rückbereiches 24 ermittelt.

## Patentansprüche

1. Absorbierender Inkontinenzartikel mit einem Hauptteil (20), bestehend aus einem Vorderbereich (22), einem Rückbereich (24) und einem in Längsrichtung (48) dazwischen liegenden zwischen den Beinen eines Benutzers zu liegen kommenden Schrittbereich (26), wobei der Hauptteil (20) einen Saugkörper (28) umfasst, und mit an den Rückbereich (24) und an den Vorderbereich (22) angrenzenden ersten Materialabschnitten (34a), welche sich in Querrichtung (38) über erste seitliche Längsränder (40a) des Hauptteils (20) hinaus erstrecken, und mit an den Rückbereich (24) und an den Vorderbereich (22) angrenzenden zweiten Materialabschnitten (34b), welche sich in Querrichtung (38) über zweite seitliche Längsränder (40b) des Hauptteils. (20) hinaus erstrecken und wobei erste und zweite Materialabschnitte (34a, 34b) den Vorderbereich (22) und den Rückbereich (24) im angelegten Zustand des Artikels miteinander verbinden, indem die beidseits an den Rückbereich angefügten Materialabschnitte Verschlusselemente (42) aufweisen, die mit einem Auftreffbereich am Hauptteil (20) lösbar haftend oder klebend zusammenwirken, wobei diese Materialabschnitte (34a, 34b) vor Ingebrauchnahme des zusammengefalteten Artikels jeweils um eine in Längsrichtung (48) verlaufende Einschlagachse (61 a, 61b) nach innen auf die körperzugewandte Seite des Hauptteils (20) eingeschlagen sind, um eine übereinander eingeschlagene Anordnung zu bilden, derart, dass der zweite Materialabschnitt (34b) zumindest bereichsweise unterhalb des ersten Materialabschnittes (34a) zu liegen kommt und diese eingeschlagene Anordnung an einer ersten Fügestelle (70) lösbar fixiert ist.

2. Inkontinenzartikel nach Anspruch 1, **dadurch gekennzeichnet, dass** ein Teilabschnitt (60) des ersten Materialabschnittes (34a) an einer ersten Fügestelle (70) an einem Teilabschnitt (60) des zweiten Materialabschnittes (34b) lösbar fixiert ist.

3. Inkontinenzartikel nach einem der Ansprüche 1 oder 2, **dadurch gekennzeichnet, dass** die jeweilige Einschlagachse (61a, 61b) innerhalb des Hauptteils (20) verläuft.

4. Inkontinenzartikel nach einem oder mehreren der vorangegangenen Ansprüche, **dadurch gekennzeichnet, dass** ein Teilabschnitt (60) des ersten Materialabschnittes (34a) an einer ersten Fügestelle (70) an der körperabgewandten Oberfläche des Hauptteils (20) in unmittelbarer Nähe des zweiten seitlichen Längsrandes (40b) des Hauptteils (20) lösbar fixiert ist.

5. Inkontinenzartikel nach einem oder mehreren der vorangegangenen Ansprüche, **dadurch gekennzeichnet, dass** die erste Fügestelle (70) durch eine einzige insbesondere punktförmige erste Fügestelle oder mehrere im Wesentlichen punktförmige erste Fügestellen ausgebildet ist.

6. Inkontinenzartikel nach einem oder mehreren der vorangegangenen Ansprüche, **dadurch gekennzeichnet, dass** die erste Fügestelle oder die ersten Fügestellen (70) der gefalteten Anordnung durch thermisch oder durch Ultraschall erzeugte Fügestellen ausgebildet ist oder sind.

7. Inkontinenzartikel nach einem oder mehreren der vorangegangenen Ansprüche, **dadurch gekennzeichnet, dass** die erste Fügestelle oder die ersten Fügestellen (70) der gefalteten Anordnung durch ein Klebemittel, insbesondere ein Heißschmelzklebemittel, insbesondere ein permanent haftendes Heißschmelzklebemittel ausgebildet ist oder sind.

8. Inkontinenzartikel nach einem oder mehreren der vorangegangenen Ansprüche, **dadurch gekennzeichnet dass** die erste Fügestelle oder die ersten Fügestellen (70) eine Klebemittelspur (80) umfassen, wobei sich die Klebemittelspur (80) in Längsrichtung (48) über 10-120 mm, insbesondere über 20-80 mm und weiter insbesondere über 30-60 mm erstreckt, und wobei sich die Klebemittelspur (80) in Querrichtung (38) über 1-20 mm, insbesondere über 1,5-15 mm und weiter insbesondere über 2-10 mm erstreckt.

9. Inkontinenzartikel nach einem oder mehreren der vorangegangenen Ansprüche, wobei die Materialabschnitte (34a, 34b) an den Windelhauptteil (20) als separate Elemente angefügt sind.

10. Inkontinenzartikel nach einem oder mehreren der vorangegangenen Ansprüche, wobei die Materialabschnitte (34a, 34b) jeweils ihrerseits wenigstens um eine in Längsrichtung (48) verlaufende Falzlinie (50, 52, 54) auf sich selbst gefaltet sind, wobei Teilabschnitte (60) der jeweiligen Materialabschnitte (34a, 34b) aufeinander zu liegen kommen.

11. Inkontinenzartikel nach Anspruch 10, wobei aufeinander gefaltete und flächenhaft aneinander liegende Teilabschnitte (60) der Materialabschnitte (34a, 34b) in dieser gefalteten Konfiguration an einer zweiten Fügestelle (62) lösbar aneinander fixiert sind.

12. Inkontinenzartikel nach Anspruch 11, **dadurch gekennzeichnet, dass** die zweite Fügestelle (62) durch eine einzige insbesondere punktförmige zweite Fügestelle oder mehrere im Wesentlichen punktförmige zweite Fügestellen ausgebildet ist.

13. Inkontinenzartikel nach mindestens einem der Ansprüche 10-12, **dadurch gekennzeichnet, dass** an einem in Querrichtung (38) das freie Ende des Materialabschnitts (34a, 34b) bildenden Teilabschnitt (60) eines jeweiligen so gefalteten Materialabschnitts (34a, 34b) ein Anfassbereich (58, 12) zum Entfalten des Materialabschnitts (34a, 34b) vorgesehen ist.

14. Inkontinenzartikel nach mindestens einem der Ansprüche 11-13, **dadurch gekennzeichnet, dass** die lösbare Fixierung an sämtlichen zweiten Fügestellen (62) beim Entfalten durch einmaliges Ziehen an einem jeweiligen Anfassbereich (58, 12) der Materialabschnitte (34a, 34b) auftrennbar ist.

15. Inkontinenzartikel nach mindestens einem der Ansprüche 11-14 **dadurch gekennzeichnet, dass** sowohl die lösbare Fixierung an sämtlichen ersten Fügestellen (70) der eingeschlagenen Anordnung als auch die lösbare Fixierung an sämtlichen zweiten Fügestellen (62) des ersten Materialabschnittes (34a) beim Entfalten durch einmaliges Ziehen an einem jeweiligen Anfassbereich (58) des ersten Materialabschnittes (34a) auftrennbar ist.

16. Inkontinenzartikel nach einem oder mehreren der Ansprüche 11-15, **dadurch gekennzeichnet, dass** die zweite Fügestelle (62) der aufeinander gefalteten Teilabschnitte (60) der Materialabschnitte (34a, 34b) aneinander durch thermisch oder durch Ultraschall erzeugte zweite Fügestellen (62) ausgebildet ist.

17. Inkontinenzartikel nach einem oder mehreren der Ansprüche 11-16, **dadurch gekennzeichnet, dass** der betreffende Teilabschnitt (60) im Anfassbereich (58, 12) keine zweiten Fügestellen (62) aufweist.

18. Inkontinenzartikel nach einem oder mehreren der vorangegangenen Ansprüche, **dadurch gekennzeichnet, dass** die Materialabschnitte (34a, 34b) eine rechteckigförmige Kontur aufweisen.

19. Inkontinenzartikel nach einem oder mehreren der Ansprüche 10-18, **dadurch gekennzeichnet, dass** ein gefalteter Materialabschnitt (34a, 34b) um wenigstens zwei Faltlinien (50, 52, 54) auf sich selbst gefaltet ist.

20. Inkontinenzartikel nach Anspruch 19, **dadurch gekennzeichnet, dass** ein gefalteter Materialabschnitt (34a, 34b) um drei oder vier Faltlinien (50, 52, 54) auf sich selbst gefaltet ist.

21. Inkontinenzartikel nach einem oder mehreren der Ansprüche 13-20, **dadurch gekennzeichnet, dass** vor der Ingebrauchnahme die jeweiligen Anfassbereiche (58, 12) in Querrichtung (38) nach außen also voneinander weg gewandt sind, so dass sie auf bequeme Weise mit der linken Hand eines Benutzers von links und mit der rechten Hand von rechts greifbar sind.

22. Inkontinenzartikel nach einem oder mehreren der Ansprüche 11-21, **gekennzeichnet durch** einen Bereich der Materialabschnitte (34a, 34b) in gefalteter Konfiguration, in dem die Anzahl oder der Flächenanteil der zweiten Fügestellen (62) oder die Haftkraft der lösbar aneinander gefügten Teilabschnitte (60) der Materialabschnitte (34a, 34b) mit der Entfernung von dem Anfassbereich in Längsrichtung (48) abnimmt.

23. Inkontinenzartikel nach einem oder mehreren der Ansprüche 11-22, **gekennzeichnet durch** einen Bereich der Materialabschnitte (34a, 34b) in gefalteter Konfiguration, in dem die Anzahl oder der Flächenanteil der zweiten Fügestellen (62) oder die Haftkraft der lösbar aneinander gefügten Teilabschnitte (60) der Materialabschnitte mit der Entfernung von dem Anfassbereich (58, 12) in Querrichtung (38) abnimmt.

24. Inkontinenzartikel nach einem oder mehreren der Ansprüche 11-23, **dadurch gekennzeichnet, dass** in einem Umkreis (71) von 1,5 cm von einem vom Anfassbereich (58, 12) am weitesten entfernten Punkt (68) der am weitesten entfernten Falzlinie (50, 54) die aneinander liegenden Teilabschnitte (60) eines jeweiligen Materialabschnittes nicht aneinander gefügt sind.

25. Inkontinenzartikel nach einem oder mehreren der Ansprüche 11-24, **dadurch gekennzeichnet, dass** in einem Abstand von 5 mm, insbesondere von 8 mm und weiter insbesondere von 10 mm von der vom Anfassbereich (58, 12) am weitesten entfernten Falzlinie (50, 54) die aneinander liegenden Teilabschnitte (60) eines jeweiligen Materialabschnittes nicht aneinander gefügt sind.

26. Inkontinenzartikel nach einem oder mehreren der Ansprüche 11-25, **dadurch gekennzeichnet, dass** die flächenhafte Erstreckung der aufeinandergefalteten und aneinander anliegenden Teilabschnitte (60) durch eine in Längsrichtung (48) verlaufende Gerade (52) in zwei annähernd gleiche Hälften teilbar ist und dass die Anzahl oder der Flächenanteil der zweiten Fügestellen (62) oder die Haftkraft der lösbar aneinander gefügten Teilabschnitte (60) eines jeweiligen Materialabschnittes in diesen zwei Hälften unterschiedlich ist.

27. Inkontinenzartikel nach Anspruch 26, **dadurch gekennzeichnet, dass** die Anzahl oder der Flächenanteil der zweiten Fügestellen oder die Haftkraft der lösbar aneinander gefügten Teilabschnitte (60) eines jeweiligen Materialabschnittes in der dem Anfassbereich (58, 12) in Querrichtung (38) zugewandten Hälfte größer ist als in der dem Anfassbereich in Querrichtung abgewandten Hälfte.

28. Inkontinenzartikel nach einem oder mehreren der vorangegangenen Ansprüche, **dadurch gekennzeichnet, dass** die Erstreckung eines angefügten Materialabschnitts (34a, 34b) in Längsrichtung (48) im Bereich der Anfügung an den Hauptteil (20) wenigstens 10 cm, insbesondere wenigstens 14 cm, insbesondere wenigstens 18 cm und weiter insbesondere wenigstens 22 cm beträgt.

29. Inkontinenzartikel nach einem oder mehreren der vorangegangenen Ansprüche, **dadurch gekennzeichnet, dass** die Erstreckung eines angefügten Materialabschnitts (34a, 34b) im entfalteten Zustand in Querrichtung (38) über den jeweiligen Längsrand (40a, 40b) des Hauptteils (20) hinaus wenigstens 10 cm, insbesondere wenigstens 15 cm, insbesondere wenigstens 18 cm, insbesondere wenigstens 22 cm beträgt.

30. Inkontinenzartikel nach einem oder mehreren der vorangegangenen Ansprüche, **dadurch gekennzeichnet, dass** die Verschlusselemente (42, 44) ihrerseits in einer gefalteten, zum Gebrauch entfaltbaren Konfiguration an den Materialabschnitten angeordnet sind.

31. Inkontinenzartikel nach einem oder mehreren der vorangegangenen Ansprüche, **dadurch gekennzeichnet, dass** die an den Hauptteil (20) angefügten Materialabschnitte (34a, 34b) von einem Vliesstoff gebildet sind.

32. Inkontinenzartikel nach einem oder mehreren der vorangegangenen Ansprüche, **dadurch gekennzeichnet, dass** die an den Hauptteil (20) angefügten Materialabschnitte (34a, 34b) weniger steif sind als der Hauptteil (20).

33. Inkontinenzartikel nach einem oder mehreren der Ansprüche 11-32, **dadurch gekennzeichnet, dass** die zweite Fügestelle oder die zweiten Fügestellen (62) beim Entfalten der Materialabschnitte (34a, 34b) durch Ziehen an dem jeweiligen Anfassbereich (58, 12) eine über den Entfaltungsvorgang gemittelte Spitzenkraft von höchstens 2,5 N, insbesondere von höchstens 2,4 N, insbesondere von höchstens 2,3 N, insbesondere von höchstens 2,2 N, insbesondere von höchstens 2,1 N und weiter insbesondere von höchstens 2,0 N entgegensetzen.

34. Inkontinenzartikel nach einem oder mehreren der Ansprüche 11-33, **dadurch gekennzeichnet, dass** die zweite Fügestelle oder die zweiten Fügestellen (62) beim Entfalten der Materialabschnitte (34a, 34b) durch Ziehen an dem jeweiligen Anfassbereich (58, 12) eine über sechs Entfaltungsvorgänge gemittelte Spitzenkraft von höchstens 2,3 N, insbesondere von höchstens 2,0 N, insbesondere von höchstens 1,8 N, insbesondere von höchstens 1,7 N, insbesondere von höchstens 1,6 N und weiter insbesondere von höchstens 1,5 N entgegensetzen.

35. Inkontinenzartikel nach einem oder mehreren der Ansprüche 11-34, **dadurch gekennzeichnet, dass** die beim Lösen der zweiten Fügestelle oder zweiten Fügestellen (62) und Entfalten eines Materialabschnitts (34a, 34b) in einem Zug erforderliche Arbeit gemittelt über sechs Entfaltungsvorgänge höchstens 120 Nmm, insbesondere höchstens 115 Nmm, insbesondere höchstens 110 Nmm, insbesondere höchstens 105 Nmm, insbesondere höchstens 100 Nmm, insbesondere höchstens 95, insbesondere höchstens 90 Nmm beträgt.

36. Inkontinenzartikel nach einem oder mehreren der vorangegangenen Ansprüche, **dadurch gekennzeichnet, dass** beim Lösen der übereinander eingeschlagenen Anordnung die erste Fügestelle oder der ersten Fügstellen (70) eine über den Ablösungsvorgang gemittelte Spitzenkraft von höchstens 3,0 N, insbesondere von höchstens 2,0 N, insbesondere von höchstens 1,5 N, und weiter insbesondere von höchstens 1,0 N entgegensetzen.

37. Inkontinenzartikel nach einem oder mehreren der vorgenannten, **dadurch gekennzeichnet, dass** beim Lösen der übereinander eingeschlagenen Anordnung die erste Fügestelle oder der ersten Fügstellen (70) eine über sechs Ablösungsvorgänge gemittelte Spitzenkraft von höchstens 2,5 N, insbesondere von höchstens 2,0 N, insbesondere von höchstens 1,5 N und weiter insbesondere von höchstens 1,0 N entgegensetzen.

38. Inkontinenzartikel nach einem oder mehreren der vorgenannten Ansprüche, **dadurch gekennzeichnet, dass** die beim Lösen der ersten Fügestelle oder ersten Fügestellen (70) in einem Zug erforderliche Arbeit gemittelt über sechs Ablösevorgänge höchstens 50 Nmm, insbesondere höchstens 40 Nmm, insbesondere höchstens 30 Nmm und weiter insbesondere höchstens 20 Nmm beträgt.

39. Inkontinenzartikel nach einem oder mehreren der vorangegangenen Ansprüche, **dadurch gekennzeichnet, dass** die erste Fügestelle oder die ersten Fügestellen (70) in einem unteren Bereich des ersten Materialabschnitts (34a) angeordnet ist oder sind.

40. Verfahren zur Herstellung eines Inkontinenzartikels nach einem der vorangehenden Ansprüche umfassend folgende Schritte:
a. Zuführen eines Windelhauptteils (20) in Längsrichtung (48) mit an den Rückbereich (24) und an den Vorderbereich (22) angrenzenden ersten Materialabschnitten (34a), welche sich in Querrichtung (38) über erste seitliche Längsränder (40a) des Hauptteils (20) hinaus erstrecken, und mit an den Rückbereich (24) und an den Vorderbereich (22) angrenzenden zweiten Materialabschnitten (34b), welche sich in Querrichtung (38) über zweite seitliche Längsränder (40b) des Hauptteils (20) hinaus erstrecken und wobei erster und zweiter Materialabschnitt wenigstens um eine erste in Längsrichtung (48) verlaufende Falzlinie (50, 52, 54) auf sich selbst gefaltet sind
b. Einschlagen der auf sich selbst gefalteten Materialabschnitte (34a, 34b) jeweils um eine in Längsrichtung (48) verlaufende Einschlagachse (61a, 61b) nach innen auf die körperzugewandte Seite des Hauptteils (20) um eine übereinander eingeschlagene Anordnung zu bilden, derart, dass der zweite Materialabschnitt (34b) zumindest bereichsweise unterhalb des ersten Materialabschnittes (34a) zu liegen kommt und
c. Fixieren dieser eingeschlagenen Anordnung an einer ersten Fügestelle (70).

41. Verfahren nach Anspruch 40, **dadurch gekennzeichnet, dass** die Einschlagachsen (61a, 61b) innerhalb des Hauptteils (20) angeordnet sind.

42. Verfahren nach Anspruch 41, **dadurch gekennzeichnet, dass** die Einschlagachsen (61a, 61b) zumindest abschnittsweise durch den Saugkörper 28 verlaufen.

## Claims

1. Absorbent incontinence article having a main part (20), comprising a front region (22), a rear region (24) and a crotch region (26), which is located between the front and rear regions in the longitudinal direction (48) and ends up located between a user's legs, wherein the main part (20) comprises a suction body (28), and having first material portions (34a), which are adjacent to the rear region (24) and the front region (22) and extend in the transverse direction (38) beyond first lateral longitudinal peripheries (40a) of the main part (20), and having second material portions (34b), which are adjacent to the rear region (24) and the front region (22) and extend in the transverse direction (38) beyond two lateral longitudinal peripheries (40b) of the main part (20), and wherein first and second material portions (34a, 34b) connect the front region (22) and the rear region (24) to one another when the article is being worn, by virtue of the fact that the material portions which are joined to either side of the rear region have closure elements (42) which interact in a releasably adhering or adhesively bonding manner with a contact region on the main part (20), wherein said material portions (34a, 34b), prior to the folded-together article being used, are each folded inwards, about a fold-in axis (61a, 61b) running in the longitudinal direction (48), onto that side of the main part (20) which is directed towards the wearer's body, this forming an arrangement in which the material portions are folded in one above the other such that at least part of the second material portion (34b) ends up located beneath the first material portion (34a), and said folded-in arrangement is fixed in a releasable manner at a first joining location (70).

2. Incontinence article according to Claim 1, **characterized in that** a sub-portion (60) of the first material portion (34a) is fixed in a releasable manner, at a first joining location (70), on a sub-portion (60) of the second material portion (34b).

3. Incontinence article according to either of Claims 1 and 2, **characterized in that** the respective fold-in axis (61a, 61b) runs within the main part (20).

4. Incontinence article according to one or more of the preceding claims, **characterized in that** a sub-portion (60) of the first material portion (34a) is fixed in a releasable manner, at a first joining location (70), on that surface of the main part (20), in the immediate vicinity of the second lateral longitudinal periphery (40b) of the main part (20), which is directed away from the wearer's body.

5. Incontinence article according to one or more of the preceding claims, **characterized in that** the first joining location (70) is formed by a single in particular point-form first joining location or more than one essentially point-form first joining locations.

6. Incontinence article according to one or more of the preceding claims, **characterized in that** the first joining location or the first joining locations (70) of the folded arrangement is or are formed by thermally or ultrasonically generated joining locations.

7. Incontinence article according to one or more of the preceding claims, **characterized in that** the first joining location or the first joining locations (70) of the folded arrangement is or are formed by an adhesive, in particular a hot-melt adhesive, in particular a permanently adhering hot-melt adhesive.

8. Incontinence article according to one or more of the preceding claims, **characterized in that** the first joining location or the first joining locations (70) comprises or comprise an adhesive track (80), wherein the adhesive track (80) extends over 10-120 mm, in particular over 20-80 mm and further particularly over 30-60 mm, in the longitudinal direction (48), and wherein the adhesive track (80) extends over 1-20 mm, in particular over 1.5-15 mm and further particularly over 2-10 mm, in the transverse direction (38).

9. Incontinence article according to one or more of the preceding claims, wherein the material portions (34a, 34b) are joined in the form of separate elements to the main part (20) of the nappy.

10. Incontinence article according to one or more of the preceding claims, wherein the material portions (34a, 34b) are each folded on themselves at least about a folding line (50, 52, 54) running in the longitudinal direction (48), wherein sub-portions (60) of the respective material portions (34a, 34b) end up located one upon the other.

11. Incontinence article according to Claim 10, wherein sub-portions (60) of the material portions (34a, 34b), said sub-portions being folded one upon the other and being in surface contact with one another, are fixed in a releasable manner on one another, in this folded configuration, at a second joining location (62).

12. Incontinence article according to Claim 11, **characterized in that** the second joining location (62) is formed by a single in particular point-form second joining location or more than one essentially point-form second joining locations.

13. Incontinence article according to at least one of Claims 10-12, **characterized in that** a sub-portion (60) of a respective material portion (34a, 34b) folded in such a way, said sub-portion forming the free end of the material portion (34a, 34b) in the transverse direction (38), has provided on it a gripping region (58, 12) for unfolding the material portion (34a, 34b).

14. Incontinence article according to one of Claims 11-13, **characterized in that** the releasable fixing at all the second joining locations (62) can be undone during the unfolding operation by virtue of a respective gripping region (58, 12) of the material portions (34a, 34b) being subjected to a single pulling action.

15. Incontinence article according to at least one of Claims 11-14, **characterized in that** both the releasable fixing at all the first joining locations (70) of the folded-in arrangement and the releasable fixing at all the second joining locations (62) of the first material portion (34a) can be undone during the unfolding operation by virtue of a respective gripping region (58) of the first material portion (34a) being subjected to a single pulling action.

16. Incontinence article according to one or more of Claims 11-15, **characterized in that** the second joining location (62) of the sub-portions (60) of the material portions (34a, 34b), said sub-portions being folded one upon the other, are formed by thermally or ultrasonically generated second joining locations (62).

17. Incontinence article according to one or more of Claims 11-16, **characterized in that** the relevant sub-portion (60) does not have any second joining locations (62) in the gripping region (58, 12).

18. Incontinence article according to one or more of the preceding claims, **characterized in that** the material portions (34a, 34b) have a rectangular contour.

19. Incontinence article according to one or more of Claims 10-18, **characterized in that** a folded material portion (34a, 34b) is folded on itself about at least two folding lines (50, 52, 54).

20. Incontinence article according to Claim 19, **characterized in that** a folded material portion (34a, 34b) is folded on itself about three or four folding lines (50, 52, 54).

21. Incontinence article according to one or more of Claims 13-20, **characterized in that**, prior to the article being used, the respective gripping regions (58, 12) are directed outwards away from one another in the transverse direction (38) such that they can be comfortably gripped from the left by a user's left hand and from the right by a user's right hand.

22. Incontinence article according to one or more of Claims 11-21, **characterized by** a region of the material portions (34a, 34b) in folded configuration in which the number or the surface-area fraction of the second joining locations (62) or the force of adherence of the sub-portions (60) of the material portions (34a, 34b), said sub-portions being joined in a releasable manner to one another, decreases as the distance from the gripping region in the longitudinal direction (48) increases.

23. Incontinence article according to one or more of Claims 11-22, **characterized by** a region of the material portions (34a, 34b) in folded configuration in which the number or the surface-area fraction of the second joining locations (62) or the force of adherence of the sub-portions (60) of the material portions (34a, 34b), said sub-portions being joined in a releasable manner to one another, decreases as the distance from the gripping region (58, 12) in the transverse direction (38) increases.

24. Incontinence article according to one or more of Claims 11-23, **characterized in that**, within a radius (71) of 1.5 cm from a point (68) of the furthest-away folding line (50, 54), said point being furthest away from the gripping region (58, 12), the abutting sub-portions (60) of a respective material portion are not joined to one another.

25. Incontinence article according to one or more of Claims 11-24, **characterized in that**, at a distance of 5 mm, in particular of 8 mm and further particularly of 10 mm, from the folding line (50, 54), which is furthest away from the gripping region (58, 12), the abutting sub-portions (60) of a respective material portion are not joined to one another.

26. Incontinence article according to one or more of Claims 11-25, **characterized in that** the surface-area extent of the abutting sub-portions (60), which are folded one upon the other, can be divided into two more or less equal halves by a straight line (52) running in the longitudinal direction (48), and **in that** the number or the surface-area fraction of the second joining locations (62) or the force of adherence of the sub-portions (60) of a respective material portion, said sub-portions being joined in a releasable manner to one another, is different in these two halves.

27. Incontinence article according to Claim 26, **characterized in that** the number or the surface-area fraction of the second joining locations or the force of adherence of the sub-portions (60) of a respective material portion, said sub-portions being joined in a releasable manner to one another, is greater in the half which is directed towards the gripping region (58, 12) in the transverse direction (38) than in the half which is directed away from the gripping region in the transverse direction.

28. Incontinence article according to one or more of the preceding claims, **characterized in that** the extent of a joined-on material portion (34a, 34b) in a longitudinal direction (48) in the region of joining to the main part (20) is at least 10 cm, in particular at least 14 cm, in particular at least 18 cm and further particularly at least 22 cm.

29. Incontinence article according to one or more of the preceding claims, **characterized in that** the extent of a joined-on material portion (34a, 34b) beyond the respective longitudinal periphery (40a, 40b) of the main part (20) in the transverse direction (38) in the unfolded state is at least 10 cm, in particular at least 15 cm, in particular at least 18 cm, and in particular at least 22 cm.

30. Incontinence article according to one or more of the preceding claims, **characterized in that** the closure elements (42, 44) are arranged in a folded configuration, which can be unfolded for use, on the material portions.

31. Incontinence article according to one or more of the preceding claims, **characterized in that** the material portions (34a, 34b), which are joined to the main part (20), are formed from a nonwoven material.

32. Incontinence article according to one or more of the preceding claims, **characterized in that** the material portions (34a, 34b), which are joined to the main part (20), are less stiff than the main part (20).

33. Incontinence article according to one or more of Claims 11-32, **characterized in that**, for the operation of unfolding the material portions (34a, 34b) by virtue of the respective gripping region (58, 12) being subjected to pulling action, the second joining location or the second joining locations (62) puts or put up a peak force, which is averaged over the unfolding operation, of not more than 2.5 N, in particular of not more than 2.4 N, in particular of not more than 2.3 N, in particular of not more than 2.2 N, in particular of not more than 2.1 N and further particularly of not more than 2.0 N.

34. Incontinence article according to one or more of Claims 11-33, **characterized in that**, for the operation of unfolding material portions (34a, 34b) by virtue of the respective gripping region (58, 12) being subjected to pulling action, the second joining location or the second joining locations (62) puts or put up a peak force, which is averaged over six unfolding operations, of not more than 2.3 N, in particular of not more than 2.0 N, in particular of not more than 1.8 N, in particular of not more than 1.7 N, in particular of not more than 1.6 N and further particularly of not more than 1.5 N.

35. Incontinence article according to one or more of Claims 11-34, **characterized in that** the work, averaged over six unfolding operations, which is necessary for releasing the second joining location or second joining locations (62) and unfolding a material portion (34a, 34b) in one pulling action is not more than 120 Nmm, in particular not more than 115 Nmm, in particular not more than 110 Nmm, in particular not more than 105 Nmm, in particular not more than 100 Nmm, in particular not more than 95 Nmm, and in particular not more than 90 Nmm.

36. Incontinence article according to one or more of the preceding claims, **characterized in that**, for the operation of releasing the arrangement in which the material portions are folded in one above the other, the first joining location or the first joining locations (70) puts or put up a peak force, averaged over the detachment operation, of not more than 3.0 N, in particular of not more than 2.0 N, in particular of not more than 1.5 N, and further particularly of not more than 1.0 N.

37. Incontinence article according to one or more of the preceding claims, **characterized in that**, for the operation of releasing the arrangement in which the material portions are folded in one above the other, the first joining location or the first joining locations (70) puts or put up a peak force, which is averaged over six detachment operations, of not more than 2.5 N, in particular of not more than 2.0 N, in particular of not more than 1.5 N and further particularly of not more than 1.0 N.

38. Incontinence article according to one or more of the preceding claims, **characterized in that** the work, averaged over six detachment operations, which is necessary for releasing the first joining location or first joining locations (70) in one pulling action is not more than 50 Nmm, in particular not more than 40 Nmm, in particular not more than 30 Nmm and further particularly not more than 20 Nmm.

39. Incontinence article according to one or more of the preceding claims, **characterized in that** the first joining location or the first joining locations (70) is or are arranged in a lower region of the first material portion (34a).

40. Method of producing an incontinence article according to one of the preceding claims, comprising the following steps:
a. a main nappy part (20) is fed in the longitudinal direction (48) with first material portions (34a), which are adjacent to the rear region (24) and the front region (22) and extend in the transverse direction (38) beyond first lateral longitudinal peripheries (40a) of the main part (20), and with second material portions (34b), which are adjacent to the rear region (24) and to the front region (22) and extend in the transverse direction (38) beyond second lateral longitudinal peripheries (40b) of the main part (20), and wherein the first and second material portions are each folded on themselves at least about a first folding line (50, 52, 54) running in the longitudinal direction (48),
b. the material portions (34a, 34b), which are each folded on themselves, are each folded inwards, about a fold-in axis (61a, 61b) running in the longitudinal direction (48), onto that side of the main part (20) which is directed towards the wearer's body, this forming an arrangement in which the material portions are arranged one above the other such that at least part of the second material portion (34b) ends up located beneath the first material portion (34a), and
c. said folded-in arrangement is fixed at a first joining location (70).

41. Method according to Claim 40, **characterized in that** the fold-in axes (61a, 61b) are arranged within the main part (20).

42. Method according to Claim 41, **characterized in that** the fold-in axes (61a, 61b) run, at least in part, through the absorbent body (28).

## Revendications

1. Article absorbant pour incontinence, comportant une partie principale (20), constituée d'une région avant (22), d'une région arrière (24) et d'une région de fourche (26) située entre elles dans la direction longitudinale (48), venant se positionner entre les jambes d'un utilisateur, la partie principale (20) comprenant un corps absorbant (28), et comportant des premières sections de matériau (34a) raccordées à la région arrière (24) et à la région avant (22) qui s'étendent dans la direction transversale (38) au-delà de premiers bords longitudinaux latéraux (40a) de la partie principale (20), et comportant des deuxièmes sections de matériau (34b) raccordées à la région arrière (24) et à la région avant (22) qui s'étendent dans la direction transversale (38) au-delà de deuxièmes bords longitudinaux latéraux (40b) de la partie principale (20), des premières et deuxièmes sections de matériau (34a, 34b) reliant l'une à l'autre la région avant (22) et la région arrière (24) dans l'état d'utilisation de l'article, les sections de matériau assemblées des deux côtés à la région arrière présentant des éléments de fermeture (42) qui coopèrent par adhésion ou adhérence de manière amovible avec une région de contact sur la partie principale (20), ces sections de matériau (34a, 34b) étant repliées vers l'intérieur avant l'utilisation de l'article plié, à chaque fois autour d'un axe de repliement (61a, 61b) s'étendant dans la direction longitudinale (48), sur le côté de la partie principale (20) tourné vers le corps, afin de former un agencement replié à chevauchement de telle sorte que la deuxième section de matériau (34b) vienne s'appliquer au moins en partie sous la première section de matériau (34a) et que cet agencement replié soit fixé de manière amovible au niveau d'un premier site de jonction (70).

2. Article pour incontinence selon la revendication 1, **caractérisé en ce qu'**une section partielle (60) de la première section de matériau (34a) est fixée de manière amovible au niveau d'un premier site de jonction (70) à une section partielle (60) de la deuxième section de matériau (34b).

3. Article pour incontinence selon la revendication 1 ou 2, **caractérisé en ce que** l'axe de repliement respectif (61a, 61b) s'étend à l'intérieur de la partie principale (20).

4. Article pour incontinence selon l'une quelconque ou plusieurs des revendications précédentes, **caractérisé en ce qu'**une section partielle (60) de la première section de matériau (34a) est fixée de manière amovible au niveau d'un premier site de jonction (70) à la surface de la partie principale (20) opposée au corps à proximité immédiate du deuxième bord longitudinal latéral (40b) de la partie principale (20).

5. Article pour incontinence selon l'une quelconque ou plusieurs des revendications précédentes, **caractérisé en ce que** le premier site de jonction (70) est réalisé par un premier site de jonction unique notamment de forme ponctuelle ou par plusieurs premiers sites de jonction de forme essentiellement ponctuelle.

6. Article pour incontinence selon l'une quelconque ou plusieurs des revendications précédentes, **caractérisé en ce que** le premier site de jonction ou les premiers sites de jonction (70) de l'agencement plié est ou sont réalisés par des sites de jonction produits thermiquement ou par ultrasons.

7. Article pour incontinence selon l'une quelconque ou plusieurs des revendications précédentes, **caractérisé en ce que** le premier site de jonction ou les premiers sites de jonction (70) de l'agencement plié est ou sont réalisés par un adhésif, en particulier un adhésif thermofusible, en particulier un adhésif thermofusible adhérant de manière permanente.

8. Article pour incontinence selon l'une quelconque ou plusieurs des revendications précédentes, **caractérisé en ce que** le premier site de jonction ou les premiers sites de jonction (70) comprennent une trace d'adhésif (80), la trace d'adhésif (80) s'étendant dans la direction longitudinale (48) sur 10-120 mm, en particulier sur 20-80 mm et plus particulièrement sur 30-60 mm, et la trace d'adhésif (80) s'étendant dans la direction transversale (38) sur 1-20 mm, en particulier sur 1,5-15 mm et plus particulièrement sur 2-10 mm.

9. Article pour incontinence selon l'une quelconque ou plusieurs des revendications précédentes, dans lequel les sections de matériau (34a, 34b) sont assemblées au niveau de la partie principale de la couche (20) en tant qu'éléments séparés.

10. Article pour incontinence selon l'une quelconque ou plusieurs des revendications précédentes, dans lequel les sections de matériau (34a, 34b) sont repliées sur elles-mêmes à chaque fois pour leur part au moins autour d'une ligne de pliage (50, 52, 54) s'étendant dans la direction longitudinale (48), des sections partielles (60) des sections de matériau respectives (34a, 34b) venant se placer les unes sur les autres.

11. Article pour incontinence selon la revendication 10, dans lequel des sections partielles (60) des sections de matériau (34a, 34b), pliées les unes sur les autres et disposées à plat les unes contre les autres, sont fixées de manière amovible les unes aux autres dans cette configuration pliée au niveau d'un deuxième site de jonction (62).

12. Article pour incontinence selon la revendication 11, **caractérisé en ce que** le deuxième site de jonction (62) est réalisé par un deuxième site de jonction unique, notamment de forme ponctuelle ou par plusieurs deuxièmes sites de jonction notamment de forme ponctuelle.

13. Article pour incontinence selon au moins l'une quelconque des revendications 10 à 12, **caractérisé en ce que** dans une section partielle (60) d'une section de matériau respective ainsi pliée (34a, 34b) formant, dans la direction transversale (38), l'extrémité libre de la section de matériau (34a, 34b), est prévue une région de saisie (58, 12) pour déplier la section de matériau (34a, 34b).

14. Article pour incontinence selon au moins l'une quelconque des revendications 11 à 13, **caractérisé en ce que** la fixation amovible au niveau de tous les deuxièmes sites de jonction (62) peut être défaite lors du dépliement en tirant une seule fois sur une région de saisie respective (58, 12) des sections de matériau (34a, 34b).

15. Article pour incontinence selon au moins l'une quelconque des revendications 11 à 14, **caractérisé en ce que** la fixation amovible au niveau de tous les premiers sites de jonction (70) de l'agencement replié ainsi que la fixation amovible au niveau de tous les deuxièmes sites de jonction (62) de la première section de matériau (34a) peuvent être défaites lors du dépliement en tirant une seule fois sur une région de saisie respective (58) de la première section de matériau (34a).

16. Article pour incontinence selon au moins l'une quelconque des revendications 11 à 15, **caractérisé en ce que** le deuxième site de jonction (62) des sections partielles (60) pliées les unes sur les autres des sections de matériau (34a, 34b) les unes contre les autres est réalisé par des deuxièmes sites de jonction (62) produits thermiquement ou par ultrasons.

17. Article pour incontinence selon l'une quelconque ou plusieurs des revendications 11 à 16, **caractérisé en ce que** la section partielle concernée (60) ne présente aucun deuxième site de jonction (62) dans la région de saisie (58, 12).

18. Article pour incontinence selon l'une quelconque ou plusieurs des revendications précédentes, **caractérisé en ce que** les sections de matériau (34a, 34b) présentent un contour de forme rectangulaire.

19. Article pour incontinence selon l'une quelconque ou plusieurs des revendications 10 à 18, **caractérisé en ce qu'**une section de matériau pliée (34a, 34b) est pliée sur elle-même autour d'au moins deux lignes de pliage (50, 52, 54).

20. Article pour incontinence selon la revendication 19, **caractérisé en ce qu'**une section de matériau pliée (34a, 34b) est pliée sur elle-même autour de trois ou quatre lignes de pliure (50, 52, 54).

21. Article pour incontinence selon l'une quelconque ou plusieurs des revendications 13 à 20, **caractérisé en ce qu'**avant l'utilisation, les régions de saisie respectives (58, 12) sont tournées vers l'extérieur dans la direction transversale (38) c'est-à-dire à l'écart l'une de l'autre, de sorte qu'elles puissent être saisies de manière aisée de la main gauche d'un utilisateur par la gauche et de la main droite par la droite.

22. Article pour incontinence selon l'une quelconque ou plusieurs des revendications 11 à 21, **caractérisé par** une région des sections de matériau (34a, 34b) de configuration pliée, dans laquelle le nombre ou la proportion de surface des deuxièmes sites de jonction (62) ou la force d'adhérence des sections partielles (60) des sections de matériau (34a, 34b) assemblées de manière amovible les unes aux autres diminue avec l'éloignement de la région de saisie dans la direction longitudinale (48).

23. Article pour incontinence selon l'une quelconque ou plusieurs des revendications 11 à 22, **caractérisé par** une région des sections de matériau (34a, 34b) de configuration pliée, dans laquelle le nombre ou la proportion de surface des deuxièmes sites de jonction (62) ou la force d'adhérence des sections partielles (60) des sections de matériau assemblées de manière amovible les unes aux autres diminue avec l'éloignement de la région de saisie (58, 12) dans la direction transversale (38).

24. Article pour incontinence selon l'une quelconque ou plusieurs des revendications 11 à 23, **caractérisé en ce que** dans un rayon (71) de 1,5 cm depuis un point (68) le plus éloigné de la région de saisie (58, 12) de la ligne de pliage la plus éloignée (50, 54), les sections partielles (60) d'une section de matériau respective situées les unes contre les autres ne sont pas assemblées les unes aux autres.

25. Article pour incontinence selon l'une quelconque ou plusieurs des revendications 11 à 24, **caractérisé en ce qu'**à l'intérieur d'une distance de 5 mm, en particulier de 8 mm et plus particulièrement de 10 mm de la ligne de pliure (50, 54) la plus éloignée de la région de saisie (58, 12), les sections partielles (60) d'une section de matériau respective situées les unes contre les autres ne sont pas assemblées les unes aux autres.

26. Article pour incontinence selon l'une quelconque ou plusieurs des revendications 11 à 25, **caractérisé en ce que** l'étendue en surface des sections partielles (60) pliées les unes sur les autres et s'appliquant les unes contre les autres peut être divisée par une droite (52) s'étendant dans la direction longitudinale (48) en deux moitiés approximativement égales et **en ce que** le nombre ou la proportion de surface des deuxièmes sites de jonction (62) ou la force d'adhérence des sections partielles (60) d'une section de matériau respective assemblées de manière amovible les unes aux autres est différent(e) dans ces deux moitiés.

27. Article pour incontinence selon la revendication 26, **caractérisé en ce que** le nombre ou la proportion de surface des deuxièmes sites de jonction ou la force d'adhérence des sections partielles (60) d'une section de matériau respective assemblées de manière amovible les unes aux autres dans la moitié tournée vers la région de saisie (58, 12) dans la direction transversale (38) est supérieur(e) à celui ou celle dans la moitié opposée à la région de saisie dans la direction transversale.

28. Article pour incontinence selon l'une quelconque ou plusieurs des revendications précédentes, **caractérisé en ce que** l'étendue d'une section de matériau assemblée (34a, 34b) dans la direction longitudinale (48) dans la région de l'assemblage à la partie principale (20) vaut au moins 10 cm, en particulier au moins 14 cm, en particulier au moins 18 cm, et encore en particulier au moins 22 cm.

29. Article pour incontinence selon l'une quelconque ou plusieurs des revendications précédentes, **caractérisé en ce que** l'étendue d'une section de matériau assemblée (34a, 34b) dans l'état déplié en section transversale (38) au-delà du bord longitudinal respectif (40a, 40b) de la partie principale (20) vaut au moins 10 cm, en particulier au moins 15 cm, en particulier au moins 18 cm, en particulier au moins 22 cm.

30. Article pour incontinence selon l'une quelconque ou plusieurs des revendications précédentes, **caractérisé en ce que** les éléments de fermeture (42, 44) sont disposés pour leur part sur les sections de matériau dans une configuration pliée, pouvant être dépliée pour l'utilisation.

31. Article pour incontinence selon l'une quelconque ou plusieurs des revendications précédentes, **caractérisé en ce que** les sections de matériau (34a, 34b) assemblées à la partie principale (20) sont formées par un matériau non tissé.

32. Article pour incontinence selon l'une quelconque ou plusieurs des revendications précédentes, **caractérisé en ce que** les sections de matériau (34a, 34b) assemblées à la partie principale (20) sont moins rigides que la partie principale (20).

33. Article pour incontinence selon l'une quelconque ou plusieurs des revendications 11 à 32, **caractérisé en ce que** le deuxième site de jonction ou les deuxièmes sites de jonction (62), lors du dépliement des sections de matériau (34a, 34b) par traction sur la région de saisie respective (58, 12) opposent une force maximale moyennée sur toute l'opération de dépliement de 2,5 N au maximum, en particulier de 2,4 N au maximum, en particulier de 2,3 N au maximum, en particulier de 2,2 N au maximum, en particulier de 2,1 N au maximum et encore en particulier de 2,0 N au maximum.

34. Article pour incontinence selon l'une quelconque ou plusieurs des revendications 11 à 33, **caractérisé en ce que** le deuxième site de jonction ou les deuxièmes sites de jonction (62), lors du dépliement des sections de matériau (34a, 34b) par traction sur la région de saisie respective (58, 12) s'opposent à une force maximale moyennée sur six opérations de dépliement de 2,3 N au maximum, en particulier de 2,0 N au maximum, en particulier de 1,8 N au maximum, en particulier de 1,7 N au maximum, en particulier de 1,6 N au maximum et encore en particulier de 1,5 N au maximum.

35. Article pour incontinence selon l'une quelconque ou plusieurs des revendications 11 à 34, **caractérisé en ce que** le travail requis lors du détachement du deuxième site de jonction ou des deuxièmes sites de jonction (62) et du dépliement d'une section de matériau (34a, 34b) en une seule traction, moyenné sur six opérations de dépliement, vaut 120 Nmm au maximum, en particulier 115 Nmm au maximum, en particulier 110 Nmm au maximum, en particulier 105 Nmm au maximum, en particulier 100 Nmm au maximum, en particulier 95 Nmm au maximum, en particulier 90 Nmm.

36. Article pour incontinence selon l'une quelconque ou plusieurs des revendications précédentes, **caractérisé en ce que** lors du détachement de l'agencement replié à chevauchement, le premier site de jonction ou les premiers sites de jonction (70) opposent une force maximale moyennée sur toute l'opération de détachement de 3,0 N au maximum, en particulier de 2,0 N au maximum, en particulier de 1,5 N au maximum, encore en particulier de 1,0 N au maximum.

37. Article pour incontinence selon l'une quelconque ou plusieurs des revendications précédentes, **caractérisé en ce que** lors du détachement de l'agencement replié à chevauchement, le premier site de jonction ou les premiers sites de jonction (70) opposent une force maximale moyennée sur six opérations de détachement de 2,5 N au maximum, en particulier de 2,0 N au maximum, en particulier de 1,5 N au maximum, et encore en particulier de 1,0 N au maximum.

38. Article pour incontinence selon l'une quelconque ou plusieurs des revendications précédentes, **caractérisé en ce que** le travail requis lors du détachement du premier site de jonction ou des premiers sites de jonction (70) en une seule traction, moyenné sur six opérations de détachement, vaut 50 Nmm au maximum, en particulier 40 Nmm au maximum, en particulier 30 Nmm au maximum et encore en particulier 20 Nmm au maximum.

39. Article pour incontinence selon l'une quelconque ou plusieurs des revendications précédentes, **caractérisé en ce que** le premier site de jonction ou les premiers sites de jonction (70) est ou sont disposés dans une région inférieure de la première section de matériau (34a).

40. Procédé de fabrication d'un article pour incontinence selon l'une quelconque des revendications précédentes, comprenant les étapes suivantes :
a. acheminement d'une partie principale de couche (20) dans la direction longitudinale (48) avec des premières sections de matériau (34a) raccordées à la région arrière (24) et à la région avant (22) qui s'étendent dans la direction transversale (38) au-delà de premiers bords longitudinaux latéraux (40a) de la partie principale (20), et avec des deuxièmes sections de matériau (34b) raccordées à la région arrière (24) et à la région avant (22) qui s'étendent dans la direction transversale (38) au-delà de deuxièmes bords longitudinaux latéraux (40b) de la partie principale (20), la première et la deuxième section de matériau étant repliées sur elles-mêmes au moins autour d'une première ligne de pliage (50, 52, 54) s'étendant dans la direction longitudinale (48),
b. repliement vers l'intérieur des sections de matériau (34a, 34b) repliées sur elles-mêmes à chaque fois autour d'un axe de repliement (61a, 61b) s'étendant dans la direction longitudinale (48) sur le côté de la partie principale (20) tourné vers le corps, afin de former un agencement replié à chevauchement de telle sorte que la deuxième section de matériau (34b) vienne s'appliquer au moins en partie sous la première section de matériau (34a) et
c. fixation de cet agencement replié au niveau d'un premier site de jonction (70).

41. Procédé selon la revendication 40, **caractérisé en ce que** les axes de repliement (61a, 61b) sont disposés à l'intérieur de la partie principale (20).

42. Procédé selon la revendication 41, **caractérisé en ce que** les axes de repliement (61a, 61b) s'étendent au moins en partie à travers le corps absorbant (28).
